# EUROPEAN PATENT APPLICATION

(11) **EP 4 458 358 A1**
(43) Date of publication of application: **06.11.2024**
(21) Application number: 23000068.9
(22) Date of filing: 02.05.2023
(51) Int. Cl.: A61K 31/4365, A61K 9/00, A61K 31/437, A61K 31/5025, A61P 11/00, A61P 17/00, A61P 43/00

(54) **PYRIDAZINE COMPOUNDS FOR ANTIFIBROTIC THERAPY**

(71) Applicant: Tietze Chemical Consulting UG, 37077 Göttingen (DE)
(72) Inventor: Voß, Edgar, 34355 Staufenberg (DE); Tietze, Lutz F., 37077 Göttingen (DE)

(57) **Abstract**

The present invention relates to a pyridazine compound of Formula I 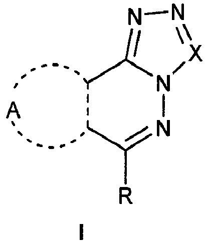 for the use in the treatment of skin and lung fibrosis, wherein the pyridazine compounds normalize overshooting collagen production.

## Description

The present invention relates to antifibrotic substances, which can be used in the treatment of fibrosis, in particular skin fibrosis or lung fibrosis.

### Technical background

### Fibrotic disease of the skin

There are a number of fibrosis in skin diseases in dermatology, all of which are characterized by the proliferation and thickening of collagen fibers. Common to all these diseases is that they have a chronic course and are generally difficult to influence therapeutically. Fibrosis can result from autoimmune or auto-inflammatory processes, post-traumatic, or circulatory disorders. Fibrosis in diseases arising from autoimmune or auto-inflammatory processes include morphea with all its subtypes, systemic scleroderma with all its subtypes, chronic graft versus host disease (GvDH), and hypertrophic scars or keloids; sclerosis in circulatory disorders arises in chronic venous insufficiency, among others.

### Localized scleroderma or morphea

Localized scleroderma or morphea is a spectrum of sclerotic diseases of the skin with possible involvement of skin-related structures such as fat tissue, muscle, joints, and bone, depending on the subtype and location. The incidence of morphea is reported to be approximately 27 per 1 million population [1, 2]. The pathogenesis of the disease is incompletely understood, but in all different forms of morphea there is an inflammatory process that leads to increased synthesis and reduced degradation of collagen fibers and thus to a chronic fibrotic connective tissue reaction [3-5]. Early in this process a very dense, predominantly lymphocytic, inflammatory infiltrate of the superficial vessels occurs, also the deep vessels may be involved, depending on the disease subtype. In some forms, e.g., eosinophilic fasciitis, the fibrotic reaction originates in the fascia. Fibroblasts are stimulated to increase synthesis of collagen fibers by various factors such as the Wnts (Wingless and Int-1) signaling pathway, oxidative stress, and reactive oxygen species but especially by transforming growth factor-β (TGF-β) and by interleukin-4 [6]. In later stages of the disease, there is a massive deposition of connective tissue structures, which is accompanied by a change in the fibril diameter of collagens and an increased variation in their thickness at the electron microscopic level [7]. In summary, there is much evidence to suggest that after an initial activation of mesenchymal cells, there is a long-lasting stimulation of these cells via an autocrine mechanism, thus leading to the activation of inflammatory and profibrotic pathways that result in excessive collagen production. Clinically, depending on the extent and depth of fibrosis, morphea is characterized by a limited, generalized, linear, deep, or mixed form, with the mixed form consisting of at least two of the aforementioned forms. The limited forms are morphea (plaque type), guttate morphea (special form of morphea), and atrophoderma idiopathica of Pasini and Pierini (special form of morphea), and the generalized form includes generalized morphea, which affects at least three anatomic areas, disabling pansclerotic morphea, a severe special form, and eosinophilic fasciitis a special form with leading affection of the fascia, the linear form includes morphea of the extremities, morphea of the "en coup de sabre" type, and progressive facial hemiatrophy (synonym: Parry Romberg syndrome) [8].

Even though it is considered a skin-limited disease, certain subtypes are associated with extracutaneous manifestations, such as musculo-articular (myositis, fasciitis and arthritis), central nervous system (headache, migraine, seizures, and epilepsy) and ocular (uveitis). In addition, they may lead to severe disfigurement, functional disability and neuro-ophthalmologic complications. Reddish or purplish roundish or linear deep-seated plaques are initially seen in most cases, which become increasingly indurated as the disease progresses due to increasing sclerosis and are often accompanied by hyperpigmentation. The epidermis is otherwise unaffected by the disease. These plaques can lead to stonewalling of joints and associated limitation of movement. The therapeutic approach addresses the inflammatory process with the use of UV light, local and systemic corticosteroids, topical tacrolimus, and systemic methotrexate, hydroxychloroquine, or mycophenolate mofetil. As with all sclerosing diseases, there is no specific therapy that can prevent the production of collagen fibers [1].

### Systemic sclerosis

Systemic sclerosis is a disease of the small arteries, microvessels, and connective tissue that initially manifests in the dermis but can also affect internal organs, particularly the digestive system, heart, lungs, and kidneys, and can lead to death. Systemic sclerosis has the highest mortality among rheumatologic diseases. Among the leading causes of death is pulmonary arterial hypertension (PAH). The 3-year survival rate of patients with systemic sclerosis and PAH is only 56%. The incidence in European countries is about 12 new cases per 1,000,000 person-years and the prevalence in is about 20 per 100,000 but may vary between countries [9, 10]. The disease affects women significantly more often than men [9, 10].

The pathogenesis of autoimmune disease is complex and not yet fully understood [11]. Initially, damage to the vasculature is probably caused by activation or apoptosis of endothelial cells due to infection, autoantibodies, or reactive oxygen species [12]. This leads to activation of the immune system, and inflammatory cytokines, especially transforming growth factor-β (TGF-β), activate fibrocytes, which cause fibrotic changes by overproduction of collagenous fibers [13]. Fibrosis occurs in the skin and subcutaneous tissue, but also affects internal organs, which can lead to loss of organ function and death.

In most cases, the disease first affects the hands, which appear to be inflamed. As the disease progresses, fibrosis of the fingers and the rest of the limb increases, that is associated with significant restriction of movement. Furthermore, the circulatory disturbance leads to necrosis, which may result in the loss of the terminal fingertips. Fibrosis in the internal organs can lead to organ-specific problems such as respiratory distress, exertional dyspnea, and the severely life-shortening pulmonary arterial hypertension, renal failure requiring dialysis, heart failure, or dysphagia and indigestion. Specific antibodies can be detected in the serum of patients with the disease. Anti-nuclear antibodies and, depending on the manifestation, other autoantibodies are almost always found.

There are different variants in the clinical presentation of this disease. For example, in limited cutaneous systemic sclerosis, the skin manifestation is limited to the face, hands, and feet and does not extend beyond the elbows or knees. Diffuse cutaneous systemic sclerosis extends well beyond these areas and may involve the trunk of the body. The overall course is much more aggressive and more often leads to death. Internal organs are also more frequently and aggressively affected in this subtype. Antibodies to topoisomerase I (Scl-70 antibodies) are typically found in affected patients. There is also systemic sclerosis without skin involvement: especially patients with anti-Ro antibodies show little or no skin involvement. Furthermore, there are overlap syndromes with signs of systemic sclerosis in combination with other rheumatic diseases such as systemic lupus erythematosus, dermatomyositis or rheumatoid arthritis.

The therapy of systemic sclerosis is primarily symptomatic and has no curative but only a slowing effect on the course of the disease. Therapeutic approaches are nonspecific immunosuppressive with UVA1 irradiation, glucocorticoids, hydroxychloroquine, azathioprine, cyclosporine or cyclophosphamide, blood flow stimulating with calcium channel antagonists, prostacyclins or prostacyclin agonists, phosphodiesterase inhibitors, or endothelin antagonists as well as stimulators of soluble guanylate cyclase. Recently, therapeutic success has been achieved with the use of the IL-6 antagonist tozilizumab and the tyrosine kinase inhibitor and angiokinase inhibitor nintedanib [14, 15] . However, there are still no drugs that can reverse or at least halt fibrosis.

### Other sclerotic autoimmune dermatoses

There are other autoimmune diseases that may not always but sometimes present with sclerosis of the skin, including systemic lupus erythematosus, dermatomyositis, and lichen sclerosus and atrophicus. Again, no specific anti-fibrotic therapy is available.

### Chronic Graft versus Host Disease

For many patients with hematologic diseases the only option of curative treatment is an allogeneic hematopoietic stem cell transplantation (allo-HSCT). However, donor lymphocytes unfortunately also lead to graft-versus-host disease (GvHD) in about 50% of the cases. The cGvHD usually occurs between 2 and 18 months after allo-HSCT. Lymphocytes target the recipient's organs and cause chronic inflammation, which, if unresponsive to corticosteroids, has a poor prognosis. cGvHD affects most commonly the skin, eyes, oral mucosa, salivary glands, genital mucosa, intestines, liver, fascia, or lungs but may strike any organ. Symptoms are similar to those seen in autoaggressive rheumatic diseases such as scleroderma, Sjögren's syndrome, lupus erythematosus, primary biliary cholangitis, bronchiolitis obliterans, immunocytopenia, and chronic immunodeficiency.

The pathogenesis of cGvHD is characterized by impaired tolerance mechanisms, involving both alloreactive and autoreactive T and B cells, as well as mechanisms of chronic inflammation with subsequent fibrosis [16-19]. Disturbances of donor-mediated peripheral tolerance are reflected here in particular in a relative deficit of antigen-specific regulatory T lymphocytes. Thus, both alloreactive and autoreactive antibodies are detectable in cGvHD. In addition, GvHD leads to damage of the thymus and bone marrow stroma, which impedes the formation of new T and B lymphocytes and, in the case of the thymus, also promotes the formation of autoreactive T cells through impaired T cell selection. Increased collagen synthesis has been described in sclerotic altered connective tissue, indicating the involvement of macrophages and fibroblasts in cGvHD[16-19].

In the skin, moderate or severe chronic sclerodermiform GvHD leads to fibrotic changes with superficial sclerosis or even deep sclerosis, where wrinkling is no longer possible. Furthermore, wound healing disorders up to ulcerations can develop here. Other symptoms are poikiloderma as well as morphea-like skin changes, increasing scaling as well as hypo- or hyperpigmentation. In addition, there is loss of skin appendages [19].

The therapy of GvDH depends on the affected organs and is primarily immunosuppressive and anti-inflammatory in design and depends on the severity of the disease [19]. Thus, from moderate disease severity, systemic therapy with corticosteroids is used, and these are combined with other immunosuppressive therapies such as cyclosporine in severe disease. An important pillar in the therapy of cutaneous GvDH is also UVA irradiation with or without psoralen and extracorporeal photopheresis. A specific therapy to reduce the overproduction of collagen fibers and thus improve the sclerosis of the skin does not exist yet.

### Dermatoliposclerosis

Dermatosclerosis may occur in patients with chronic lymphedema, which occurs after infections, radiation, traumatic events, tumors and most often after surgery or in patients with chronic venous insufficiency. Chronic venous insufficiency is one of the most common of all diseases. Venous disease is diagnosed in approximately 30% of adults in industrialized nations [20-22]. The disease is caused by congestion of blood in the peripheral larger and smaller veins of the lower extremity. Chronic edema, usually occurring in the setting of chronic venous insufficiency (CVI) or leg ulcers, can lead to chronic hardening of the subcutaneous adipose tissue or fascia. This condition is referred to as dermatoliposclerosis. The exact pathogenesis is not fully understood, but inflammatory processes are important for the development of the clinical picture in addition to valvular insufficiencies. Leukocytes trapped in the microvessels appear to cause inflammation, which leads to migration of additional immune cells such as macrophages. These produce metalloproteinases that may lead to further tissue destruction. This creates intracellular fugues and can lead to the formation of inflammatory edema, which further exacerbates the process. In chronic edema, there is an obstruction of oxygen supply, as well as stimulation of fibroblasts, which is accompanied by increased formation of collagen. The collagen formation leads in the sense of a vicious circle to a further deterioration of the tissue trophism and favors the development of ulcerations. In dermatoliposclerosis, the skin is cemented to the fascial support and can no longer be lifted in folds. Often, the entire distal third of the lower leg is constricted like a tank [22, 23]. Clinical symptoms manifest primarily on the skin of the lower extremity. Initially, edema develops in the pretibial ankle area. In the course, brown pigmentation, atrophic changes up to connective tissue transformation and the final stage of a leg ulcer occur. Furthermore, there is pronounced itching. The severity of CVI is classified according to the CEAP classification. This classification includes six stages (C0-C6) that represent the progressive severity of chronic venous disease. Chronic venous disease refers to the early stages (C0-C3), while chronic venous insufficiency refers specifically to the later and more severe stages (C3-C6). **C0:** No visible or palpable signs of venous disease **C1:** telangiectasias and reticular veins. **C2:** varicose veins, **C3:** edema, **C4a:** pigmentation or eczema **C4b:** lipodermatosclerosis or atrophy blanche, **C5:** healed venous ulcer, **C6:** active venous ulcer: symptomatic, including pain, tension, skin irritation, heaviness, and muscle cramps, as well as other complaints attributed to venous dysfunction [20-23].

Therapy includes care and treatment of the skin as well as therapy of the underlying CVI. Here, refatting of the skin, topical corticosteroids and calcineurin inhibitors, medical compression therapy, and hydroxyethyl rutoside are used; as oral therapy, pentoxifylline, vein-activating medication such as calcium channel blockers, angiotensin.converting enzyme inhibitors, and angiotensin receptor blockers have been used [22]. Surgical interventions such as foam sclerotherapy, endovenous thermal ablation and phlebectomy are also important therapeutic options. There is no therapy available to inhibits the proliferation of collagen fibers to prevent dermatoliposclerosis.

### Hypertrophic scars, keloids and folliculitis keloidalis nuchae

This is a group of diseases that have fibrotic elevated dermal lesions in common and often occur after injury to the skin. These lesions may be itchy and painful, and may impair function due to associated contraction. Furthermore, the findings lead to a reduction in quality of life for aesthetic reasons [24, 25]. Although scars are a part of everyday life, exact data on the incidence and prevalence of hypertrophic scars and keloids based on published studies cannot be reliably obtained, but they occur frequently in everyday clinical practice. The pathogenesis is not fully understood. Excessive amounts of collagen fibers type 1 produced by hyperproliferative fibroblasts are observed in the extracellular matrix. The extent of the quantity of collagen fibers seems to be proportional to an underlying inflammatory response, so immunological factors such as the presence of mast cells, macrophages, lymphocytes, various cytokines, chemokines and others have been associated with the occurrence and severity of fibrotic changes [25]. Available therapies include topical or intralesional application of corticosteroids, cryotherapy, micro needling, laser therapy, pressure and traction therapy, and intralesional injection of fluorouracil and bleomycin, as well as radiotherapy, but none of the methods of scar therapy can achieve scar reduction or functional and/or cosmetic improvement in all cases. A first line treatment method cannot be standardized for scars because too many variables such as localization, age and type of scar, or genetic disposition influence the development and regression of scars. Often a combination of different treatment methods is required and even these do not often lead to appealing results. Prophylaxis that prevents overproduction of collagen fibers in high-risk patients after traumatic events such as surgical procures is not available [26].

### Sclerosis in wound healing

Sclerosis can also develop during the wound healing process. In particular, sclerotic processes occur after burns or extensive skin and soft tissue injuries, and after inflammation. Wounds generally heal more poorly in people with diabetes. High sugar levels promote inflammation and prevent the breakdown of destroyed tissue. Circulatory disorders and an increased susceptibility to wound infections can also delay wound healing. This in turn can promote sclerotic scars.

In sclerotic scars, the connective tissue below the skin surface increases and thickens. This causes the affected tissue to shorten and harden. It pulls the scar in like a band, causing the skin at this point to literally shrink. The shortened tissue impairs joint function and mobility. As a result, contractures can form. The therapy is based on plastic surgical techniques (e.g. Z-plasty, W-plasty) in combination with laser therapy and scar ointments. A therapy that directly inhibits the excessive production of collagen fibers to positively influences these scars does not exist.

### Other sclerotic disease

Furthermore, there are sclerosis of the skin or subcutaneous tissue in the context of other diseases, which occur less frequently and which may be caused by a wide variety of different factors such as:
- Infectious causes: f.e. lyme disease.
- Drug- or toxin-induced: f.e. toxic oil syndrome or eosinophilic myalgia syndrome (L-tryptophan), nephrogenic systemic fibrosis after administration of gadolinium-containing contrast agents, scleroderma-like clinical pictures caused by chemical noxae (vinyl chloride, silicon dioxide)
- Pseudoscleroderma due to drugs: phenobarbital, D-penicillamine, gold preparations, hepatitis B vaccine, captopril, spironolactone, allopurinol, bleomycin, doxetaxel, cisplatin, pentazocine, (centrally acting analgesic), cocaine, appetite suppressants.
- Metabolism-induced: f.e. scleroedema adultorum, scleromyxedema, amyloidosis, porphyria cutanea tarda, phenylketonuria, mucopolysaccharidoses, scleroderma-like skin changes in diabetes mellitus or necrobiosis lipoidica
- Induced by foreign materials: f.e. Silicone implant, polyvinyl chloride disease
- Idiopathic- or immunologically induced: Progeria adultorum (Werner syndrome), progeria infantilis, sclerofascia, fibrosis, oral submucosal, Nephrogenic fibrosing dermopathy), stiff-skin syndrome
- Paraneoplastic induced: Bronchial carcinoma, metastatic carcinoid
- Immunocytic lymphoma.
- Physically induced: Scleroderma-like changes in radiatio of breast carcinoma.

The pathogenesis of the above diseases is different and complex. The therapy options are limited and frequently therapy attempts often frustrate. For none of these mentioned diseases is there a specific therapy that prevents or reduces the proliferation of collagen fibers. We see a very large area of application in dermatology for specific cutaneous, intralesional, or oral use of drugs that reduce the formation of collagen fibers in sclerosing diseases.

### Fibrotic diseases of the lung

In several parenchymal lung diseases a fibrosis will develop over the course of the disease. Since these pathologic abnormalities predominate in the lung interstitium, the disorders are termed interstitial lung diseases (ILDs) [27]. ILDs are assigned to many disease categories mostly on the basis of a known underlying disease (e.g., pulmonary fibrosis associated with rheumatoid arthritis), an inciting agent (e.g., pneumoconiosis), or the absence of a known cause (e.g., Idiopathic pulmonary fibrosis ) [27].

The collective disease burden of ILD is significant with a prevalence of 76.0 cases per 100,000 people in Europe and 74.3 cases per 100,000 in the United States [28].

ILDs can be divided into ILDs caused by primary diseases such as sarcoidosis, Langerhans-cell granulomatosis, eosinophilic pneumonia, lymphangioleiomyomatosis, and pulmonary alveolar proteinosis; by environmental exposures, including pneumoconiosis due to inhalation of inorganic substances and hypersensitivity pneumonitis mostly related to inhalation of organic particles, by drugs, illicit drugs, or irradiation; by rheumatoid arthritis, systemic sclerosis, idiopathic inflammatory myopathy, and primary Sjögren's disease; and by idiopathic interstitial pneumonia including idiopathic pulmonary fibrosis, idiopathic nonspecific interstitial pneumonia, and other, less common entities [27].

The prognosis of the diseases is bleak, especially if the fibrosis is progressive, the dieseases will ultimately result in organ failure, causing respiratory symptoms, limited exercise capacity, an impaired quality of life, and an increased risk of death [28, 29].

Among the ILDs the idiopathic pulmonary fibrosis (IPF) is the most common. IPF is characterized by an imaging and pathological pattern of usual interstitial pneumonia without an identifiable cause. It occurs more commonly in men than in women and in older patients. IPF is a chronic and irreversible disease, usually progressing to respiratory failure with a median interval between diagnosis and death of 3 years [27, 30]. Beside IPF also systemic sclerosis-ILD, as previously described, rheumatoid arthritis- ILD, sarcoidosis, fibrotic (stage IV), chronic fibrotic hypersensitivity pneumonitis and unclassifiable fibrotic ILD are with fibrosis associated lung diseases [27]. They are generally characterized by a younger mean age at presentation and a more balanced sex ratio. The variable underlying pathological features of other ILDs, with fibrosis generally less prominent than inflammatory infiltration, may lead to less severe outcomes, as compared with IPF, however also in the other ILDs progressive fibrosis frequently occur.

Pulmonary fibrosis is also a known complication of acute respiratory distress syndrome (ARDS), and there are similarities in the fibroproliferative response and risk factors between lung fibrosis in the context of ARDS and lung fibrosis in the context of other diseases [31].

The fibrosis of the different ILDs are probably causes by in inadequate response of the lung tissue to disease specific triggers, which set off exaggerated cascades of inflammatory and fibrotic responses, leading to downstream fibrotic tissue remodeling and extracellular-matrix deposition, which in turn 8 perpetuate fibrosis formation, forming scar tissue and modifying its mechanical properties [28]. Scarring is accompanied by a strong increase in the tissue stiffness and an overall thickening of the alveolar septae leading to a strong impairment of lung function and eventually resulting in the death of the patient [13, 27, 31-33]. A genetic predisposition to an enhanced susceptibility to pulmonary fibrosis has also been discovered [34].

The main presenting symptoms are cough, progressive exertional dyspnea, and exercise limitation. The diagnosis is often delayed by several months or even years. A thorough history, including environmental exposures, medication use, and extrapulmonary signs, should be taken [30]. On chest auscultation, fine crackles (also called Velcro rales or crepitations) are indicative of fibrosis [35]. Serologic testing is recommended, including for antinuclear antibodies and anti- citrullinated peptide antibodies as well as high-resolution computed tomographic [30].

In patients with pulmonary fibrosis, testing typically shows a restrictive lung-function pattern (decreased forced vital capacity [FVC], normal or increased ratio of forced expiratory volume in 1 second to FVC, decreased total lung capacity, and low residual volume), together with a decreased diffusing capacity of the lung for carbon monoxide. However, normal lung function does not rule out the presence of pulmonary fibrosis [30].

Untreated IPF will lead to respiratory failure in virtually every patient and even with treatment the prognosis is grim. Of all patients with a diagnosis of pulmonary fibrosis other than IPF still less than half of the patients will despite treatment progress to respiratory failure with worsening respiratory symptoms, a decline in lung function, a decreased quality of life, and a risk of early death, independent of the classification of the ILD [29].

The treatment in most cases of ILDs other than IPF, immunomodulation with the use of glucocorticoids, immunosuppressive therapy, or both is indicated and is generally used as first-line therapy if there is a suspicion of inflammation-driven disease [27]. For patients with IPF, treatment with pirfenidone or nintedanib is recommended. Nintedanib has been approved by the Food and Drug Administration (FDA) and the European Medicines Agency (EMA) for patients with SSc-ILD and for patients with chronic fibrosing ILDs with a progressive phenotype. Nintedanib could effectively suppress inflammation, angiogenesis, and fibroblast activation but in cannot be used for the reversal of the progressive fibrosis [14, 36]. It is therefore not associated with an improvement in function but reduces the decline in FVC by about half. Nintedanib is a pan-kinase inhibitor of angiogenic receptors such as VEGFR, PDGFR, FGFR, and others, a profile that is typically used to block angiogenesis in tumor patients, especially non-small-cell lung cancer where the drug is approved for. Thereby, this antiproliferative compound suffers from serious side-effects, e.g. internal bleeding, abdominal pain, vomiting, and diarrhea, that are well known and limiting the anticancer treatment. Its use is mainly justified by the high mortality rate and utmost desperation seen in patients suffering from the mentioned lung fibroses. The adverse event that most frequently led to permanent discontinuation of nintedanib was progression of idiopathic pulmonary fibrosis [37].

Pirfenidone is attenuates profibrotic pathways by targeting several molecules including transforming growth factor α and interleukin 6 [38] and mediate inhibition of MUC1 bioactivation [39]. It reduces disease progression in patients with progressive, unclassifiable, fibrotic ILD [36, 40, 41]. Pirfenidone has to be taken thrice daily in high doses comes with numerous obstacles. Several liver complications such as transaminase enhancement, drug-induced liver injury, and drug-drug interactions by inhibition of CYP1A2 were reported. The compound comes with severe phototoxity that can lead to the Stevens-Johnson-Syndrom (SJS) and toxic epidermal necrolysis (TEN). All these, in part life-threatening, complications need intensive monitoring. Lung transplantation offers a chance for long-term survival but not everyone with pulmonary fibrosis is a good transplant candidate and organs suitable for transplantation are scarce.

Since none of the currently available medications are able to reverse the ILDs or even to reliably inhibit progression of the diseases especially in case of IPF, there is a huge medical need for fibrotic ILD. In general the medical need for the treatment of devastating lung fibroses is extremely high. Such treatment should stop the extensive ECM secretion of activated fibroblasts by specifically reversing the aggressive phenotype back to normal maintenance activity rather than inducing apoptosis and general cell toxicity. Surprisingly we found that compounds of the general Formula (I) were able to achieve this goal by downmodulating overshooting fibroblast action without the toxic cellular action involved with current treatment options.

### Summary of the invention

Surprisingly the present inventors found that compounds of the general Formula (I) 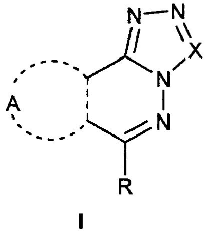 normalize the overshooting collagen production in fibroblasts cells that were taken from patients with severe fibrotic diseases. Such cells are permanently activated and provide an excellent in vitro model of such fibrotic diseases. They provide a more realistic model because they do not need any kind of artificial activation by addition of growth factors such as TGFβ. Although it is more challenging to stop elevated production of collagen in such cells, we unexpectedly found that compounds of the general Formula (I) reverse this aggressive fibroblast phenotype without toxic action. Examples **47** to **50** show the biological properties of the pyridazine compounds of the present invention.

Compounds of general Formula (I) have high value for the prevention and treatment of fibrotic diseases with life-threatening outcome or severe reduction of life quality. Examples of such diseases are skin fibrosis and lung fibrosis, in particular skin fibrosis.

The present invention is therefore directed to pyridazine compound (I) 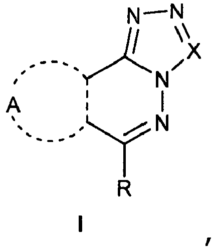 wherein
- R: is selected from the group consisting of H, D, OR³, and C₁₋₄-alkyl, in particular methyl;
- X: is N or CR¹, wherein the nitrogen and carbon atom are aromatic ring atoms;
- A: is selected from a group CR²=CR²-CR²=CR², S-CR²=CR² or
CR²-S-CR² , thus forming either a benzopyridazine (phthalazine) or a thienopyridazine compound, wherein the carbon atoms are part of an aromatic ring system and wherein 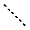 as part of the pyridazine ring denotes a single bond in case of CR²-S-CR² or a double bond in case of CR²=CR²-CR²=CR² or S-CR²=CR²; and wherein 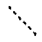 as part of ring A denotes a single bond in case of CR²=CR²-CR²=CR² or S-CR²=CR² or a double bond in case of CR²-S-CR²
and wherein
R¹ is selected from the group consisting of H, D, CD₃, CHD₂, CH₂D, CF₃, CHF₂, CH₂F, CDF₂, CD₂F, C₁₋₄-alkyl, optionally substituted with OH, halogen, OR³;
R² in case that X denotes a CR¹ group is selected from the group consisting of H, D, F, Cl, methyl, CH₂F, CHF₂, CF₃, OCF₃, OCH₃, OCD₃; and in case that X denotes N as an aromatic ring atom group R² is selected from the group consisting of H, D, F, Cl, methyl, CH₂F, CHF₂, CF₃;
R³ is selected from the group consisting of C₁₋₄-alkyl;
for use in a method of treatment or prevention of skin or lung fibrosis.

The present invention is further directed to pharmaceutical compositions comprising a pyridazine compound or a mixture of pyridazine compounds each having the structure (I) 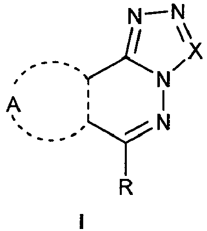 as defined in claim 1 and a pharmaceutically acceptable carrier, preferably for use in the treatment of skin and lung fibrosis.

### Detailed description of the invention

The present invention relates to new pyridazine compounds of structure (I) as such, in particular to their use in treatment of skin or lung fibrosis:

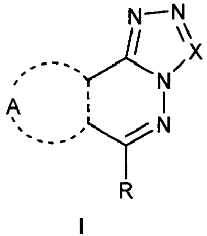

In general, compounds of Formula **I** can be prepared by methods well known in the art (Druey and Rinigier, Helv. Chim. Acta, 34 (1951), 195.; Zimmer, Kokosa, and Shah, J. Org. Chem. 40 (1975), 2901). If **X** represents nitrogen (tetrazolopyridazine compounds), two typical approaches are shown in scheme I. Thus, a chloropyridazine of Formula **II** is reacted with a metal azide in a dipolar aprotic solvent at higher temperature (method A). Preferred metal azide is sodium azide and favorable conditions are DMF as solvent and temperatures of about 120°C for several hours. Method A is exemplified by examples **2** and **35.**

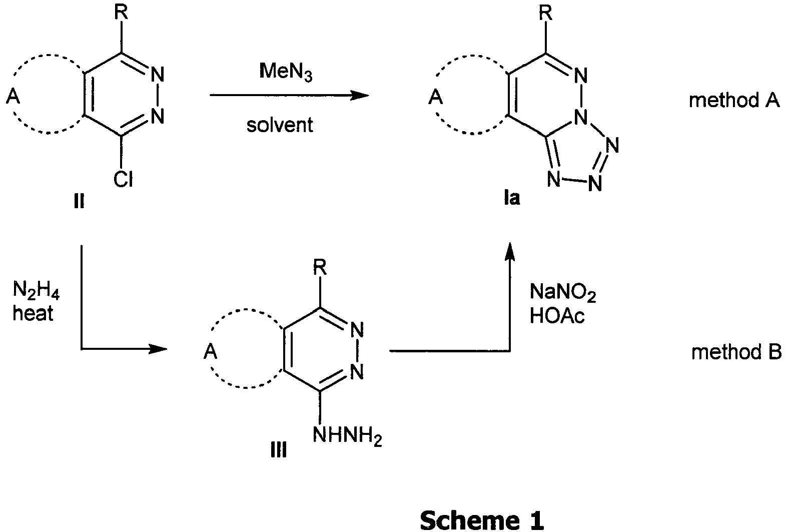

Another approach uses a hydrazine compound of Formula **III** which is reacted with nitrous acid that is formed *in situ* from sodium nitrite under acidic conditions (Badr 1984), (method B). Preferred conditions are diluted acetic acid and slight excess of nitrite at 0°C to room temperature (r.t.).

If **X** in Formula **I** represents a substituted carbon atom several known methods can be applied (Druey 1951). Typical approaches are summarized in scheme **2.** Thus compounds of Formula **III** can be reacted with reactive carboxylic acids (method C1) or an activated carboxylic acid derivative (method C2, C3). Reactive carboxylic acids are lower alkyl carboxylic acids (e.g. formic, acetic or propionic acid) or carboxylic acids containing an electron with-drawing substituent at the alpha position e.g. halogen or oxygen). Preferred conditions are using the carboxylic acid as solvent and heating to reflux for several hours.

Activated carboxylic acid derivatives include anhydrides (Badr, El-Sherief, El-Naggar, and Mahgoub, J. Heterocycl. Chem. (1984), 21, 471.) (method C2), formic acid esters, ortho esters (method C3) or carboxylic acid derivatives with an appropriate leaving group. Preferred conditions are refluxing with excess of anhydride or reaction with ortho ester at r.t in an alcoholic solvent.

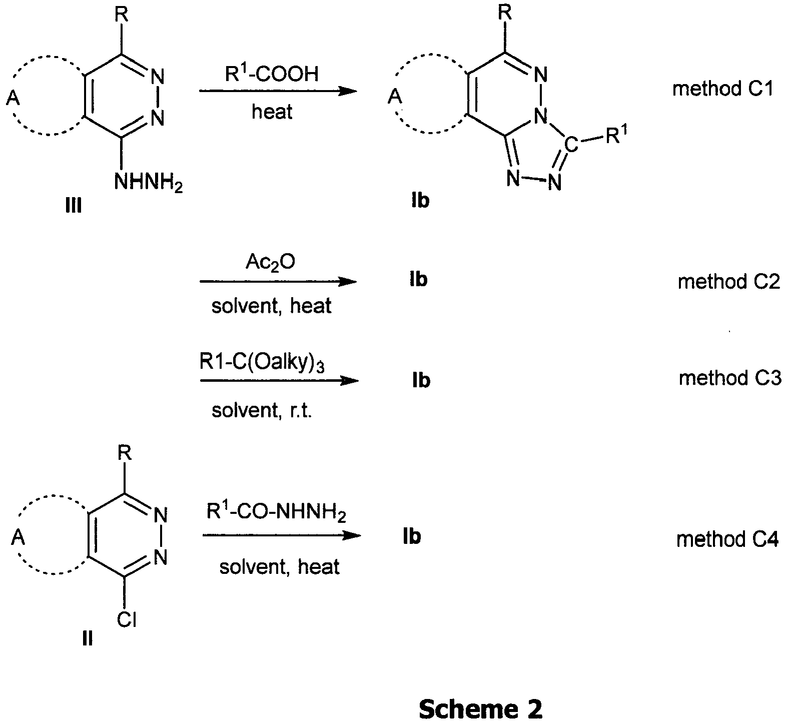

Alternatively, a chloroazine of Formula **II** can be reacted with a carboxylic acid hydrazide at higher temperature in aprotic solvents (method C4). Preferred conditions are at 100-120°C with typical solvents as DMF or dioxane.

In case that A represent a fused heterocyclic ring (Het) such as thiophene or furan the required intermediates for the synthesis of compounds of the general formula **I** can be obtained by a directed ortho-metallation (DoM) reaction. Either thiophene carboxylic acids or furan carboxylic acids are treated with strong base such as butyllithium. The lithiated heterocycles can be quenched with DMF to yield ortho-formyl acids (method D1) or alternatively with dry ice (carbon dioxide) to give the corresponding ortho-dicarboxylic acids (method D2) (scheme 3).

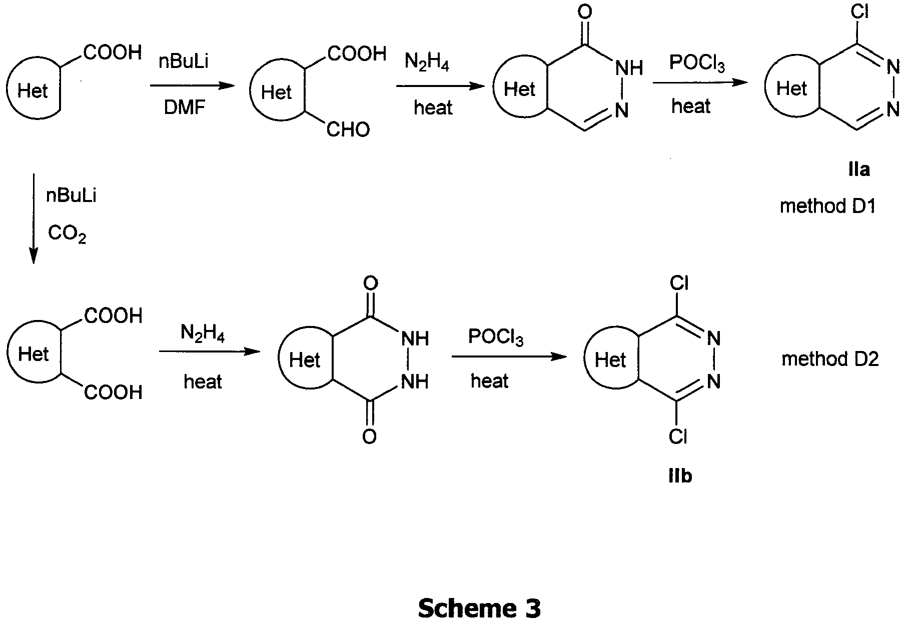

The obtained ortho-formyl carboxylic acids are easily converted to pththalazinones by heating with aqueous hydrazine to give the chlorophthalazines **IIa** by treatment with phosphorus oxychloride (method D1). An analogous procedure is used for the preparation of dichloroazines **IIb** (method D2).

In a further method substituted benzoic acids are reacted with dibromomethane aided by palladium catalysis to give the corresponding phthalide (scheme 4). The latter after bromination with NBS, followed by reaction with hydrazine to yield the corresponding phthalazinones that are converted to chloroazines of formula **IIc** (method E).

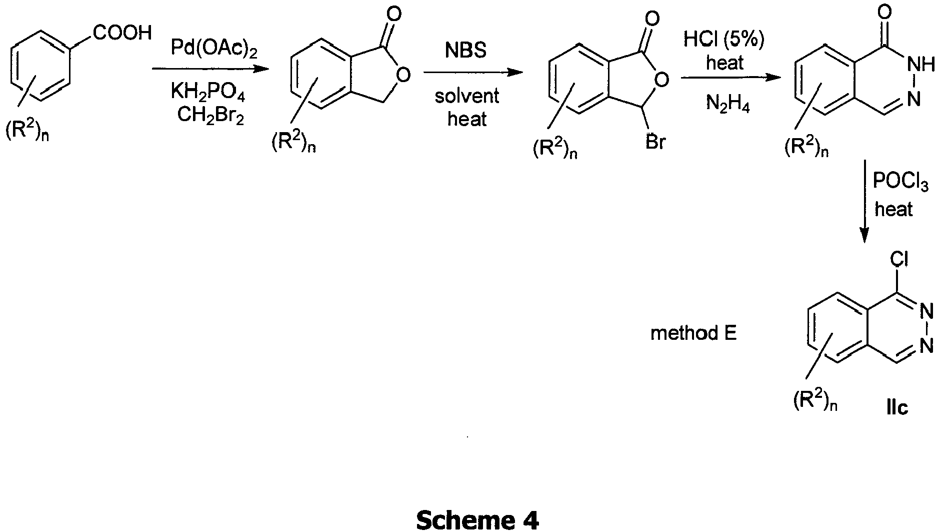

In a first embodiment, in general Formula (I), R is selected from any one of the group of the group consisting of H, D, OR³, and C₁₋₄-alkyl. That is, if R = H, H is connected via a covalent bond to the carbon atom of the ring: -H, similarly, when R = D, D is connected via a covalent bond to the carbon atom of the ring: -D, similarly, when R = OR³, OR³ is connected via a covalent bond to the carbon atom of the ring, similarly for any group of the groups from which R can be selected. Preferably, R is selected from the group consisting of H, CH₃, OCH₃, OC₂H₅, OCH(CH₃)₂. Further preferably, R is selected from the group consisting of H and D. In the most preferred embodiment R is H.

The term "alkyl" as used herein refers straight chain/linear, or branched hydrocarbon substituents, Examples of alkyl groups include methyl, ethyl, propyl, butyl, isopropyl, sec-butyl, isobutyl, *tert-butyl.*

In general Formula (I), 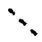 denotes a single bond or a double bond. While a single bond is directly adjacent to either double bond, a double bond cannot be selected to be directly adjacent to a neighboring double bond.

As shown in general Formula (I), A is selected such that the aromatic ring consists of five or six members.

In general Formula (I), R¹ is selected from the group consisting of H, D, CH₃, CD₃, CHD₂, CH₂D, CF₃, CHF₂, CH₂F, CDF₂, CD₂F, C₁₋₄-alkyl, optionally substituted with OH, halogen, OR³. Preferably, R¹ is selected from the group consisting of H, D, CH₃, CD₃, CF₃. That is, if R¹ = H, H is connected via a covalent bond to the carbon atom of the ring: -H, similarly for any group of the groups from which R¹ can be selected.

In general Formula (I), in case that X denotes CR¹, R² is selected from the group consisting of H, D, F, Cl, methyl, CH₂F, CHF₂, CF₃, OCH₃, OCD₃. Preferably, R² is selected from the group consisting of H, F, Cl and OCH₃. That is, if R² = H, H is connected via a covalent bond to the carbon atom of the ring: -H, similarly for any group of the groups from which R² can be selected.

In general Formula (I), in case that X denotes N, R² is selected from the group consisting of H, D, F, Cl, methyl, CH₂F, CHF₂, CF₃. Preferably, R² is selected from the group consisting of H, F, Cl. That is, if R² = H, H is connected via a covalent bond to the carbon atom of the ring: -H, similarly for any group of the groups from which R² can be selected.

In general Formula (I) wherein one R² group is selected from the list defined above, the remaining R² groups are hydrogens.

In general Formula (I) wherein two R² groups are selected from the list defined above, the remaining R² groups are hydrogens.

In the context of the present invention, all "embodiments" described herein can be combined with each other. That is, by way of example, if one "embodiment" defines R¹, it can be combined with a further "embodiment" which defines R² or any other substituent.

The pyridazine compound shown by general Formula (I) is used for the treatment of fibrosis. Examples of fibrosis are skin fibrosis that leads to a very bad quality of life and lung fibrosis that is a life-threatening disease with inferior treatment options. The pyridazine compound shown by general Formula (I) can be used for the treatment of skin fibrosis and lung fibrosis as per os application by using a single compound or by using mixtures of any two or more thereof. Optionally, the pyridazine compound is administered in combination with excipients selected from inorganic or organic excipients or combinations thereof. Inorganic excipients include calcium phosphates, calcium carbonate, calcium sulfate, halides, metallic oxides, talc. Organic excipients include carbohydrates such as sugars, sugar alcohols, starch, cellulose such as cellulose ethers, cellulose esters, carboxymethylcellulose, microcrystalline cellulose, petrochemicals, povidones, acrylic polymers.

In a further embodiment the pyridazine compound shown by general Formula (I) can be used as topical treatment for skin fibrosis. In another embodiment the pyridazine compound shown by general Formula (I) can be used by inhalation for treatment of lung fibrosis.

Structures formed by general Formula (I) encompass:

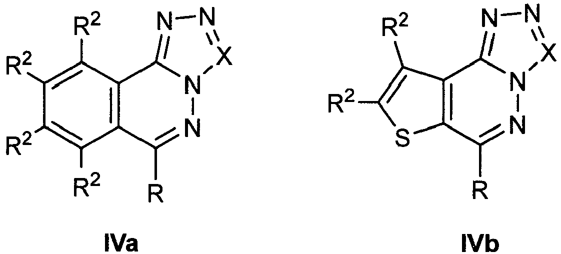

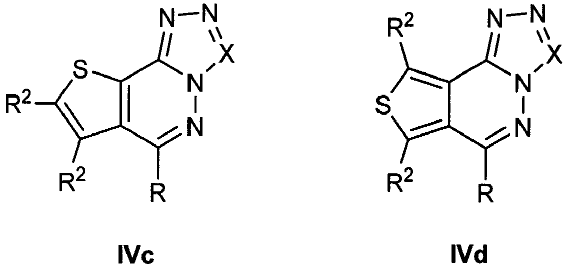

The structures preferably formed by selecting A can be shown by general Formulae (IVa) and (IVb-d), wherein general Formula (IVa) shows a six-membered ring formed by A expressing a benzopyridazine (phthalazine) and wherein general Formulae (IVb-d) show a sulfur-containing five-membered ring formed by A expressing a thienopyridazine.

And wherein R² in Formulae IVa-d are the same as defined in general Formula (I).

It is noted that hydrogen atoms are not shown in Formula (I). Furthermore, any atom in the general Formula (I) can be substituted by any of its isotopes. That is, any hydrogen atom in general Formula (I) (not shown in Formula (I)) can be substituted (partially or fully) by deuterium D. Preferred positions for the substitution of hydrogen with deuterium are as defined in the claims. For example, R can be selected from the group comprising hydrogen and deuterium as defined in the claims. Furthermore, R¹ can be selected from the group comprising hydrogen and deuterium as defined in the claims. Furthermore, any alkyl group of R¹ can optionally be fully or partially be substituted by deuterium instead of hydrogen. Furthermore, R² can be selected from the group comprising hydrogen and deuterium as defined in the claims. Optionally, R¹ = CD₃ or R = D and/or R = D.

Furthermore, any carbon atom in the general Formula (I) can be substituted (partially or fully) by any of its isotopes, preferably ¹³C. Furthermore, any nitrogen atom in the general Formula (I) can be substituted (partially or fully) by ¹⁵N.

In a second embodiment, R is selected from the group consisting of H, OR³, methyl, ethyl, propyl, isopropyl, butyl, isobutyl, *sec-*butyl, tert butyl, and cyclopropyl.

In a further embodiment, R¹ is selected from the group consisting of H, D, CD₃, CHD₂, CH₂D, CF₃, CHF₂, CH₂F, methyl, ethyl, propyl, isopropyl, butyl, isobutyl, *sec-*butyl, *tert*-butyl and cyclopropyl, optionally substituted by OH, Halogen or OR³, optionally, wherein R¹ is selected from the group consisting of H, methyl, CD₃, CF₃, CH₂OH, ethyl, CH₂OCH₃ and cyclopropyl.

In a further embodiment, R³ is selected from the group consisting of methyl, ethyl, propyl, isopropyl, butyl, isobutyl, sec-butyl and tert-butyl.

In a further embodiment, X is selected from the group consisting of N and CR¹ and wherein R¹ is selected from the group consisting of H, methyl, CH₂F, CD₂F, CHF₂, CDF₂, and CF₃, preferably, wherein X is CR¹ and R is H or D.

In a further embodiment R = H, X = CH₃ and one or two of CR² are independently selected from C-F or C-Cl.

In a further embodiment R = H, X = N and one or two of CR² are independently selected from C-F or C-Cl.

In a further embodiment R = H, X = CH and A is selected to form the moiety CH=CH-CH=CH.

In a further embodiment, R=H, X= C-CH₃ or N and A is selected to form the moiety CH=CH-CH=CH.

In a further embodiment, R=H, X= C-CH₃ or N and A is selected to form the moiety CH-S-CH.

In a further embodiment, X is C-CH₃, R is H, and A is forming the group CR²=CR²-CR²=CR² wherein one R² group is selected from the group consisting of F, Cl and OCH₃ and wherein the remaining R² groups are hydrogens.

In a further embodiment, X is C-CH₃, R is H, and A is forming the group CR²=CR²-CR²=CR² wherein two R² groups are independently selected from the group consisting of F, Cl and OCH₃ and wherein the remaining R² groups are hydrogens. In a further embodiment, the pyridazine compound (I) is selected from the group consisting of:

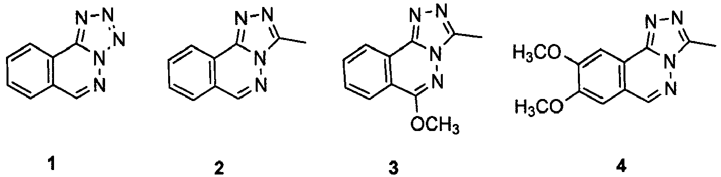

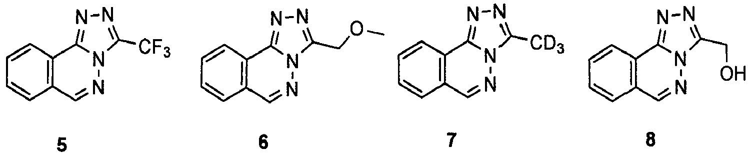

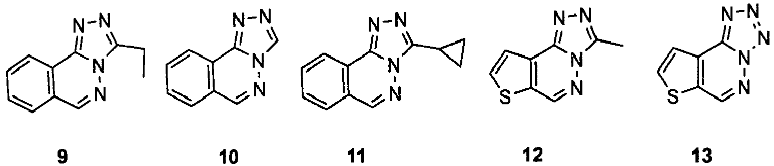

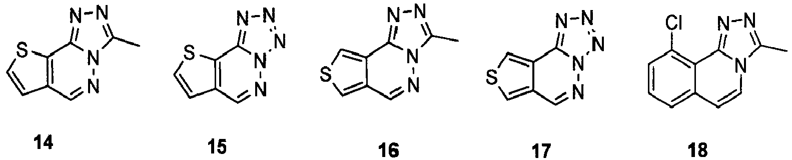

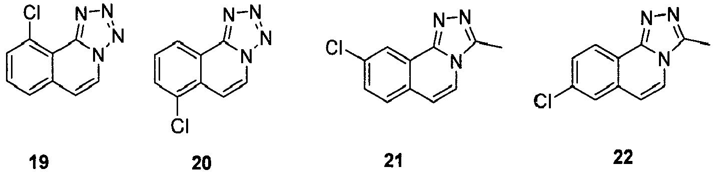

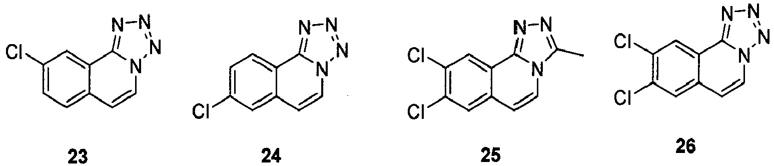

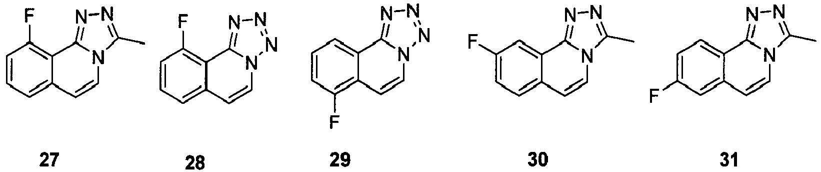

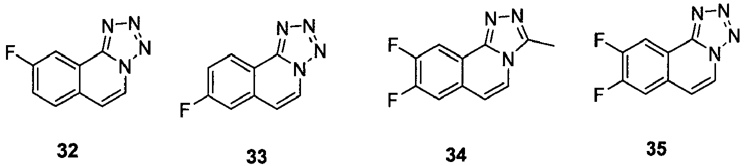

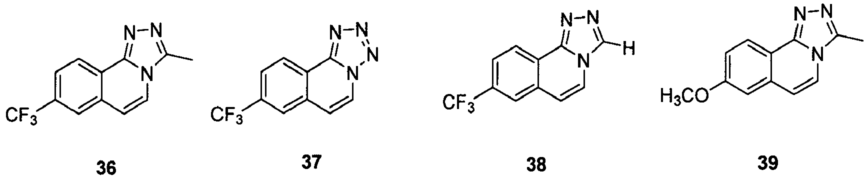

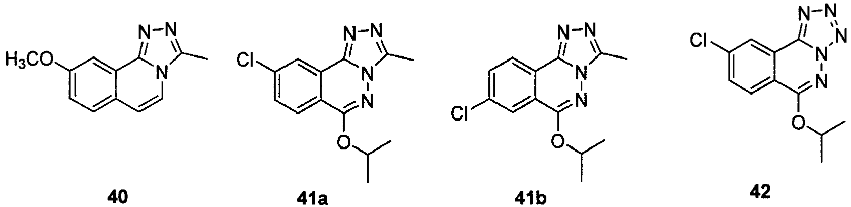

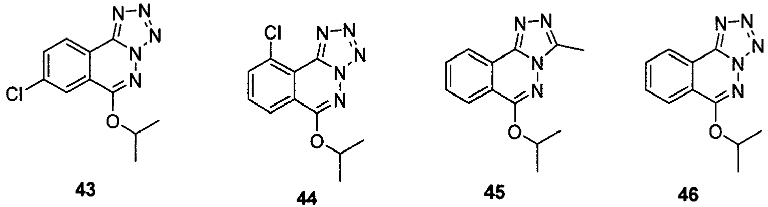

The pyridazine compound shown by general Formula (I) is used in a method of treatment or prevention of fibrosis. In a further embodiment, fibrosis is selected from the group consisting of skin fibrosis and lung fibrosis.

In a further embodiment, one or more of the carbon (C) atoms and nitrogen (N) atoms in the embodiments disclosed herein can be ¹³C isotopes, and N atoms can be ¹⁵N isotopes, wherein it is possible to have compounds with ¹³C isotopes and ¹⁵N isotopes. Furthermore, changing the isotopic composition of H/D, C and N can be applied in the assessment of drug pharmacology to determine the pharmacokinetic profile or mode of action of a drug substance. Secondly, stable isotopes may be used as internal standards for the assessment of drug concentration or its metabolites in blood or urine by analytical quantification LC-MS, HPLC-MS, LC-MS-MS or HPLC-MS-MS methods.

In a further embodiment, provided is a pharmaceutical composition comprising an pyridazine compound or a mixture of pyridazine compounds each having the structure (I) 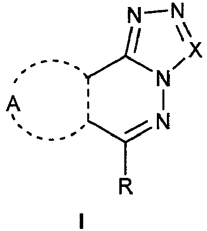 as defined in claim 1 and a pharmaceutically acceptable carrier for use in the treatment of skin fibrosis or lung fibrosis.

In a further embodiment, the pharmaceutical composition is an oral solid dosage form and/or the pyridazine compound is present in an effective amount. Effective amounts of the pyridazine compound range from 5 to 500 mg, preferably 10 to 200 mg. The compounds according to this invention and their pharmaceutically acceptable salts can be used as medicaments, e.g. in the form of pharmaceutical compositions. The pharmaceutical compositions can be administered orally, e.g. in the form of tablets, coated tablets, dragees, hard and soft gelatine capsules, solutions, emulsions or suspensions. The administration can, however, be parenterally, e.g. in the form of injection solutions.

The needed pharmaceutical compositions can be obtained by processing the compounds according to this invention with pharmaceutically acceptable, inorganic or organic carriers. Lactose, corn starch or derivatives thereof, talc, stearic acids or its salts and the like can be used, for example, as such carriers for tablets, coated tablets, dragees and hard gelatine capsules. Suitable carriers for soft gelatin capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like. The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances. Determination of a therapeutically effective amount is within the skill in the art. The therapeutically effective amount or dosage of a compound according to this invention can vary within wide limits and may be determined in a manner known in the art. Such dosage will be adjusted to the individual requirements in each particular case including the specific compound(s) being administered, the route of administration, the condition being treated, as well as the patient being treated. In general, in the case of oral or parenteral administration to adult humans weighing approximately 70 kg, a daily dosage of about 5 mg to about 500 mg, preferably from about 10 mg to about 200 mg, can be considered as a therapeutically effective amount, although the upper limit may be exceeded when indicated. The daily dosage can be administered as a single dose or in divided doses, or for parenteral administration, it may be given as continuous infusion.

The above-mentioned pharmaceutical compositions can be obtained by processing the compounds according to this invention with pharmaceutically acceptable, inorganic or organic carriers. Lactose, corn starch or derivatives thereof, talc, stearic acids or its salts and the like can be used, for example, as such carriers for tablets, coated tablets, dragees and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

A pharmaceutical composition can comprise e.g. the following:
a) Tablet Formulation (Wet Granulation):

| **Item** | **Ingredients** | **mg/tablet** | | | |
|---|---|---|---|---|---|
| 1. | Compound of formula (1) | 5 | 25 | 100 | 500 |
| 2. | Lactose Anhydrous DTG | 125 | 105 | 30 | 150 |
| 3. | Sta-Rx 1500 | 6 | 6 | 6 | 30 |
| 4. | Microcrystalline Cellulose | 30 | 30 | 30 | 150 |
| 5. | Magnesium Stearate | 1 | 1 | 1 | 1 |
| | Total | 167 | 167 | 167 | 831 |

b) Capsule Formulation:

| **Item** | **Ingredients** | **mg/capsule** | | | |
|---|---|---|---|---|---|
| 1. | **C**ompound of formula (I) | 5 | 25 | 100 | 500 |
| 2. | Hydrous Lactose | 159 | 123 | 148 | --- |
| 3. | Corn Starch | 25 | 35 | 40 | 70 |
| 4. | Talc | 10 | 15 | 10 | 25 |
| 5. | Magnesium Stearate | 1 | 2 | 2 | 5 |
| | Total | 200 | 200 | 300 | 600 |

As used herein, the term "a therapeutically effective amount" of a compound means an amount of compound that is effective to prevent, alleviate or ameliorate symptoms of disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage will be adjusted to the individual requirements in each particular case including the specific compound(s) being administered, the route of administration, the condition being treated, as well as the patient being treated. In general, in the case of oral or parenteral administration to adult humans weighing approximately 70 kg, a daily dosage of about 5 mg to about 500 mg, preferably from about 10 mg to about 200 mg, can be considered as a therapeutically effective amount, although the upper limit may be exceeded when indicated. The daily dosage can be administered as a single dose or in divided doses, or for parenteral administration, it may be given as continuous infusion.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention are contemplated. Supplementary active compounds can also be incorporated into the compositions.

The compounds according to the present invention may exist and be administrated in the form of their pharmaceutically acceptable salts or esters. The term "pharmaceutically acceptable salt" refers to conventional acid-addition salts that retain the biological effectiveness and properties of the compounds of formula I and are formed from suitable non-toxic organic or inorganic acids. Sample acid-addition salts include those derived from inorganic acids such as hydrochloric acid, sulfuric acid, and those derived from organic acids such as p-toluenesulfonic acid, naphthalenesulfonic acid, naphthalenedisulfonic acid, methanesulfonic acid, ethanesulfonic acid and the like. The chemical modification of a pharmaceutical compound (i.e. a drug) into a salt is a technique well known to pharmaceutical chemists to obtain improved physical and chemical stability, hygroscopicity, flowability and solubility of compounds (cf, e.g. Bastin, R. J., et al., Organic Proc. Res. Dev. 4 (2000) 427-435). Preferred are the pharmaceutically acceptable salts, which are formed with p-toluenesulfonic acid, naphthalenesulfonic acid, naphthalenedisulfonic acid, methanesulfonic acid, sulfuric acid, and hydrochloric acid.

In a preferred embodiment, the pyridazine compound as defined in the claims is administered in the form of a tablet.

In a further preferred embodiment, the pyridazine compound as defined in the claims can be administered especially in the case of skin- and lung-fibrosis in the following ways:
1. Topically as ointments, creams, foams, gels, lotions, and sprays
2. Intralesionally as subcutaneous and intradermal injections
3. Systemically as orally, intravenously (parenterally), sublingually, and inhalatively or using a plaster

### Examples

### Synthesis of compounds

### Example 1

### Tetrazolor5,1-alphthalazine 1

370 mg (5.35 mmol) sodium nitrite were dissolved in a minimum amount of water and added at 0°C under stirring to a solution of 982 mg (5.00 mmol) hydralazine hydrochloride and 450 mg (5.50 mmol) sodium acetate in 10 ml 2N acetic acid. A precipitate is formed immediately. After 30 min. the precipitate was isolated by filtration and washed with little ice-cold water. Drying in vacuo at 40°C gave 784 mg (86%) **1.** Extraction of the combined filtrates with ethyl acetate gave additional 60 mg (6%) of **1.** ¹H-NMR (300MHz, CDCl₃): δ = 8.03 (td, J= 7.7, 1.3 Hz, 1H), 8.14 (mc, 2H), 8.76 (d, J= 7.9 Hz, 1H), 8.96 (s, 1H, 6-H).

### Example 2

### 3-Methyl-1,2,4-triazolor[3,4-a]phthalazine 2

1.01 ml (5.50 mmol) triethyl orthoacetate were added to a suspension of 983 mg (5.00 mmol) hydralazine hydrochloride in 10 ml methanol and stirred at r.t. After 15 min. the suspension cleared and a new precipitation started. After 1h additional 10 ml of methanol were added, stirring continued for 1h and the mixture then evaporated. The residue was taken up in 40 ml water and pH adjusted to 7 with solid NaHCO₃. The formed precipitate was isolated by filtration, washed with ice-cold water, and sucked dry. Drying at 50°C in vacuo gave 833 mg (90%) of **2.** Combined filtrates were extracted with 40 ml ethyl acetate to yield additional 30 mg (3%) of **2.** ¹H-NMR (300MHz,): δ = 2.83 (s, 3H, CH₃), 7.79 (ddd, *J* = 8.3, 7.4, 1.2 Hz, 1H), 7.93 (td, *J=* 8.7, 1.2 Hz, 1H), 7.99 (td, *J=* 7.6, 1.2 Hz, 1H), 8.61 (s, 1H), 8.63 (dd, *J* = 9.1, 1.2 Hz, 1H).

### Example 3

### 6-Methoxy-3-methyl-1,2,4-triazolo[3,4-a]phthalazine 3

### 6-Chloro-3-methyl-1,2,4-triazolo[3,4-a]phthalazine (i)

A mixture of 2.58 g (13.0 mmol) 1,4-dichlorophthalazine, 50 ml ethanol and 5.0 ml of 55% hydrazine were heated to reflux for 30 min. After cooling to r.t. a felty precipitate was sucked off and washed twice with ice-cold ethanol. After washing with 10 ml t-butyl methyl ether and 10 ml pentane, the residue was dried in vacuo. Yield 2.23 g (84%) of 1-chloro-4-hydrazinylphthalazine.

An analytical sample (211 mg, 1.08 mmol) of the above was treated with 0.9 ml (1.12 mmol) of 1.25 M HCl in methanol, evaporated and dried in vacuo to give the hydrochloride of 1-chloro-4-hydrazinylphthalazine. ¹H-NMR (300MHz, D₂O): δ= 7.97-8.29 (m, 4H).

Acetic anhydride (0.5 ml, 5 mmol) was added to a suspension of 195 mg (1.00 mmol) 1-chloro-4-hydrazinylphthalazine in 6.0 ml dioxane and the mixture heated for 1h to reflux. After evaporation the residue was treated with 10 ml of ice water and adjusted to pH 7 by addition of sodium bicarbonate. After extraction with 2x 25 ml dichloromethane and evaporation the remaining solid was purified (silica, dichloromethane/methanol 20:1). Yield after drying in vacuum 198 mg 6-chloro-3-methyl-1,2,4-triazolo[3,4-a]phthalazine (i). ¹H-NMR (300MHz, CDCl₃): δ = 2.82 (s, 3H, CH₃), 7.86 (ddd, *J=* 8.3, 7.4, 1.2Hz, 1H), 8.00 (td, *J=* 7.6, 1.2 Hz, 1H), 8.26(ddd, *J=* 8.2, 1.3, 0.6 Hz, 1H), 8.67 (ddd, *J=* 8.0, 1.3, 0.7 Hz, 1H).

Compound (i) (0.050 g, 0.22 mmol, 1.0 equiv.) was dissolved in MeOH (3 mL), then Pd-C (10%) (50 mg) was added. The reaction mixture was stirred for 24 h at room temperature and then filtered through Celite. From the filtrate the solvent was evaporated and the remaining solid dried in vacuo (41 mg, 84%) **3.** ¹H-NMR (400 MHz, CDCl₃): δ = 8.56 (ddd, *J=* 8.0, 1.3, 0.7 Hz, 1H), 8.14 (ddd, *J=* 8.1, 1.3, 0.7 Hz, 1H), 7.87 (ddd, *J =* 8.0, 7.3, 1.2 Hz, 1H), 7.72 (ddd, *J* = 8.1, 7.3, 1.3 Hz, 1H), 4.18 (s, 3H), 2.74 (s, 3H). ¹³C-NMR (126 MHz, CDCl₃): δ= 158.4, 147.8, 142.6, 133.7, 130.4, 125.1, 124.7, 123.22, 118.69, 55.30, 9.92. HRMS (ESI): cal. mass C₁₁H₁₀N₄O [M-1]⁻ 213.0782, found [M-1]⁻ 213.0781.

### Example 4

### 8,9-Dimethoxy-3-methyl-1,2,4-triazolo[3,4-a]phthalazine 4

A mixture of 57 mg (0.25 mmol) 1-chloro-6,7-dimethoxypthalazine, 100 mg (1.3 mmol) acetic acid hydrazide, and 6.0 ml dioxane were heated to reflux for 12 h. After evaporation the residue was purified by column chromatography on silica (dichloromethane/methanol 20:1) to give 38 mg (61%) of **4.** ¹H-NMR (300MHz, CDCl₃): δ = 2.80 (s, 3H, CH₃), 4.04 (s, 3H, OCH₃), 4.10 (s, 3H, OCH₃), 7.22 (s, 1H), 7.96 (s, 1H), 8.48 (s, 1H).

### Example 5

### 3-Trifluoromethyl-1,2,4-triazolo[3,4-a]phthalazine 5

237 mg (1.21 mmol) hydralazine hydrochloride and 5.0 ml trifluoroacetic acid were heated to 70°C for 4 h. The mixture was evaporated and 5.0 ml water added. After adjustment to pH 5 the product was extracted with 2x 20 ml dichloromethane. Drying (Na₂SO₄) and evaporation gave 273 mg (92%) yellow solid that was purified by column chromatography on silica (dichloromethane/methanol 50:1). Yield 263 mg (89%) colorless **5.** ¹H-NMR (300MHz, CDCl₃): δ = 7.97 (dd, *J* = 8.4, 7.0 Hz, 1H), 8.07 (d, *J=* 8.0 Hz, 1H), 8.09 (td, *J=* 7.2, 1.4 Hz, 1H), 8.79 (dd, *J =* 7.9, 1.2 Hz), 8.84 (s, 1H).

### Example 6

### 3-Methoxymethyl-1,2,4-triazolo[3,4-a]phthalazine 6

100 mg (mmol) 1-Chlorophthalazine and 200 mg (mmol) methoxyacetic acid hydrazide were suspended in 5.0 ml dioxane and heated to reflux for 2 h. The reaction mixture was evaporated and the residue distributed between 20 ml water and 20 ml dichloromethane. The water phase was extracted with 20 ml dichloromethane and the combined organic phases washed with 10 ml water. After drying over sodium sulphate and evaporation, the residue was purified over a silica column (eluent: ethyl acetate). Yield 112 mg (82%) of **6.** ¹H-NMR (300MHz, CDCl₃): δ = 3.49 (s, 3H, OCH₃), 5.05 (s, 2H, CH₂O), 7.82 (td, J= 7.5, 1.2 Hz), 7.95 (d, J= 7.5 Hz, 1H), 7.96 (td, *J=* 7.5, 1.1Hz, 1H), 8.67 (dd, *J=* 7.1, 1.2 Hz, 1H), 8.68 (s, 1H).

### Example 7

### 3-D₃-Methyl-1,2,4-triazolo[3,4-a]phthalazine 7

314 mg (1.60 mmol) Hydralazine hydrochloride and 2.5 ml acetic acid-d₄ were heated to 120°C for 6 h. After evaporation 5.0 ml water was added and the pH brought to 7 by addition of NaHCO₃. Extraction with 2x 50 ml dichloromethane, drying (Na₂SO₄), and evaporation gave a yellow solid containing d3-acetic acid. Purification was achieved by two-fold column chromatography on silica (first column dichlormethane/methanol 30:1, second column ethyl acetate) yielded 236 mg (75%) **7** as colorless solid. ¹H-NMR (300MHz, CDCl₃): δ = 7.75(t, *J=* 7.4 Hz, 1H), 7.88 (m, 2H), 8.56 (d, *J=* 7.4 Hz, 1H), 8.57 (s, 1H).

### Example 8

### 1,2,4-Triazolor3,4-alphthalazin-3-yl-methanol 8

302 mg (1.83 mmol) 1-chlorophthalazine and 349 mg (3.87 mmol) hydroxyacetic acid hydrazide were added to 5.0 ml dioxane and heated to reflux for 4 h. After evaporation the residue was taken up in 10 ml water and the resulting suspension extracted with 7x with 50 ml dichloromethane. After drying (Na₂SO₄) and evaporation the residue was purified by column chromatography over silica (dichloromethane/methanol 20:1). Yield 349 mg (90%) **8** as colorless solid. ¹H-NMR (300MHz, CDCl₃): δ = 4.95 (s, 2H), 5.70 (br, s, 1H, OH), 7.94 (td, J= 7.7, 1.2 Hz, 1H), 8,06 (td, *J* = 7.6, 1.2 Hz, 1H), 8.22 (d, *J* = 7.8 Hz, 1H), 8.51 (d, *J* = 7.9 Hz, 1H), 9.09 (s, 1H).

### Example 9

### 3-Ethyl-1,2,4-triazolo[3,4-a]phthalazine 9

A solution of 376mg hydralazine hydrochloride (1.91 mmol) in 3.0 ml propionic acid is heated to 120°C for 4 h. After cooling to r.t. 20 ml water was added and the solution neutralized by addition of NaHCO₃. Extraction with 20 ml dichloromethane, drying over Na₂SO₄ evaporation gave a yellow-brown residue. Purification by column chromatography over silica (dichloromethane → dichloromethane/methanol 20:1) gave 38 mg of **9.** ¹H-NMR (300MHz, CDCl₃): δ = 1.50(t, J= 7.6 Hz, 3H, CH₃), 3.23 (q, *J* = 7.6 Hz, 2H, CH₂), 7.78 (ddd, *J* = 7.2, 6.8, 1.2 Hz, 1H), 7.91 (d, *J* = 7.4 Hz, 1H), 7.92 (t, *J* = 7.9 Hz, 1H), 8.52 (s, 1H), 8.53 (d, *J* = 7.9 Hz, 1H).

### Example 10

### 1,2,4-Triazolo[3,4-a]phthalazine 10

0.55 ml (3.0 mmol) triethyl orthoformate were added to a solution of 500 mg (2.54 mmol) hydralazine hydrochloride in 5.0 ml methanol. Stirring at r.t. was continued for 2 h. A fine crystalline precipitate was formed within 1h. After evaporation 5 ml water was added and pH adjusted to pH 5 with solid NaHCO₃. After extraction with 3x 20 ml dichloromethane the organic phase was dried (Na₂SO₄) and evaporated. Drying in vacuo gave 401mg (87%) of **10.** ¹H-NMR (300MHz, CDCl₃): δ = 7.83 (ddd, J= 8.2, 7.3, 1.2 Hz, 1H), 7.96(d, *J=* 7.9 Hz, 1H), 7.98 (td, *J=* 7.5, 1.2 Hz, 1H), 8.64 (s, 1H), 8.68 (dd, J= 7.6, 1.2 Hz, 1H), 9.04 (s, 1H).

### Example 11

### 3-Cyclopropyl-[1,2,4]triazolo[3,4-a]phthalazine 11

178 mg NaHCO₃ were added to a solution of 180 mg of cyclopropane carboxylic acid in 20 ml methanol and evaporated. To the residue were added 188 mg hydralazine hydrochloride, 500 mg cyclopropane carboxylic acid, and 10 ml dioxane. The mixture was heated to reflux for 6 h. After twice column chromatography on silica (first: ethyl acetate / hexane 2:1; second dichloromethane / methanol 50:1) 18 mg of **11** were obtained.

¹H NMR (300 MHz, CDCl₃) δ 8.63 (d, J= 7.9 Hz, 1H), 8.60 (s, 1H), 7.92 (t, J= 7,5 Hz 1H), 7,91 (d, J= 7,7 Hz, 1H), 7.79 (dd, J= 8.5, 6.6 Hz, 1H), 2.50 (tt, *J=* 8.5, 5.0 Hz, 1H), 1.39 (dt, J= 6.4, 3.3 Hz, 2H), 1.21 (dt, J= 8.5, 3.3 Hz, 2H).

### Example 12

### 3-Methylthieno[3,2-d][1,2,4]triazolo[4,3-b]pyridazine 12

### 2-Formylthiophene-3-carboxylic acid (ii)

*n*-BuLi (15.60 mL, 39.01 mmol, 2.5 mol/L, 2.5 equiv.) was added dropwise to a stirring solution of commercially available thiophene-3-carboxylic acid (2.0 g, 15.60 mmol, 1.0 equiv.) in dry THF (40 mL) at -78 °C. After 2 h, DMF (6 mL, 78.03 mmol, 5.0 equiv.) was added. After another 1 hour, the cooling bath was removed, and the mixture stirred at ambient temperature for 16 h. HCl (aq., 1 M, 60 mL) was added. The mixture was extracted with ethyl acetate (3 × 30 mL), dried (Na₂SO₄) and concentrated in vacuo. The resulting yellow solid (ii)(yield 2.20 g, 91%) was used without further purification. ¹H NMR (300 MHz, CDCl₃) δ 10.60 (s, 1H), 7.72 - 7.65 (m, 2H). HRMS (ESI): cal. mass C₆H₄O₃S [M+1]⁺ 156.9954, found [M+1]⁺ 156.9951, cal. mass C₆H₄O₃SNa [M+Na]⁺ 178.9773, found [M+Na]⁺ 178.9772.

### Thieno[2,3-d]pyridazin-4(5H)-one (iii)

A round-bottomed flask was equipped with a stir bar and reflux condenser. To the flask was added the product of (ii) (1.9 g, 12.16 mmol, 1.0 equiv.), hydrazine (55%) (1.52 g, 30.41 mmol, 2.5 equiv.), and EtOH (5 mL) and refluxed for 3 h. The reaction was cooled to room temperature and concentrated by rotary evaporation. Water (20 mL) was added and the filtrate was separated from the insoluble solids. The aqueous layer was concentrated by rotary evaporation to give a pale-yellow solid. The solid was dried in a vacuum oven overnight at 50 °C. Desired compound (xii) was obtained (1.56 g, 84% yield). ¹H NMR (500 MHz, DMSO) δ= 12.84 (s, 1H), 8.57 (s, 1H), 8.04 (d, J= 5.2 Hz, 1H), 7.63 (d, *J=* 5.1 Hz, 1H). ¹³C NMR (126 MHz, DMSO) δ= 158.38, 140.71, 135.85, 133.50, 133.08, 123.93. HRMS (ESI): cal. mass C₆H₄N₂OS [M+1]⁺ 153.0117, found [M+1]⁺ 153.0119, cal. mass C₆H₄N₂OSNa [M+Na]⁺ 174.9937, found [M+Na]⁺ 174.9936.

### 4-Chlorothieno[2,3-d]pyridazine (iv)

A round-bottomed flask was equipped with a stir bar and reflux condenser. To the flask was added the product of (iii) (1.3 g, 8.54 mmol, 1.0 equiv.), phosphorus oxychloride (8 mL, 85.43 mmol, 10.0 equiv.), and pyridine (1.4 mL, 17.08 mmol, 2.0 equiv.) and refluxed for 24 h. The reaction was cooled to r.t. and poured over ice and then neutralized with NaHCO₃. The mixture was separated and the aqueous layer was extracted with chloroform (4 × 30 mL). The organic layers were combined, dried (Na₂SO₄) and concentrated by rotary evaporation to give a yellow solid (iv) (0.73 g, 50% yield). ¹H NMR (400 MHz, CDCl₃) δ 9.58 (d, J= 0.8 Hz, 1H), 7.96 (d, *J* = 5.3 Hz, 1H), 7.63 (dd, *J=* 5.3, 0.8 Hz, 1H). ¹³C NMR (101 MHz, CDCl₃) δ 151.33, 145.69, 140.39, 136.00, 134.23, 122.44. HRMS (EI): cal. mass C₆H₃ClN₂S [M]⁺ 169.9700, found [M]⁺ 169.9698.

### 3-Methylthieno[3,2-d][1,2,4]triazolo[4,3-b]pyridazine 12

Compound (iv) (75 mg, 0.43 mmol, 1.0 equiv.) and acetic acid hydrazide (65 mg, 0.87 mmol, 2.0 equiv.) were refluxed in dioxane (3 mL) for 3 h. The reaction mixture was extracted with dichloromethane (4 × 20 mL). The organic phase was dried with Na₂SO₄ and evaporated. The crude product was purified by column chromatography using dichloromethane give amorphous solid **12** (72 mg, 87%). ¹H NMR (400 MHz, CDCl₃) δ 8.73 (d, *J=* 0.7 Hz, 1H), 8.07 (dd, *J=* 5.2, 0.8 Hz, 1H), 8.00 (d, J= 5.3 Hz, 1H), 2.84 (s, 3H). ¹³C NMR (101 MHz, CDCl₃) δ 147.65, 142.51, 140.03, 134.99, 131.53, 130.12, 122.73, 10.28. HRMS (ESI): cal. mass C₈H₆N₄S [M+1]⁺ 191.0386, found [M+1]⁺ 191.0388, cal. mass C₈H₆N₄SNa [M+Na]⁺ 213.0205, found [M+Na]⁺ 213.0208.

### Example 13

### Tetrazolo[1,5-b]thieno[3,2-d]pyridazine 13

Compound (iv) from example 12 (70 mg, 0.41 mmol, 1.0 equiv.) was dissolved in DMF (1 mL), then sodium azide (32 mg, 0.48 mmol, 1.2 equiv.) was added. The reaction mixture heated at 120°C for 3 h. The reaction was cooled to r.t. and poured into water. The mixture was separated and the aqueous layer was extracted with chloroform (4 × 20 mL). The organic layers were combined, dried (Na₂SO₄) and concentrated by rotary evaporation to give a yellow amorphous solid **13** (70 mg, 97% yield). ¹H NMR (300 MHz, DMSO) δ 9.55 (s, 1H), 8.63 (d, J= 5.2 Hz, 1H), 8.19 (d, J= 5.2 Hz, 1H). ¹³C NMR (75 MHz, DMSO) δ 143.62, 141.48, 140.39, 134.60, 130.26, 122.42. HRMS (ESI): cal. mass C₆H₃N₅S [M+1]⁺ 178.0182, found [M+1]⁺ 178.0184, cal. mass C₆H₃N₅SNa [M+Na]⁺ 200.0001, found [M+Na]⁺ 200.0006.

### Example 14

### Tetrazolo[1,5-b]thieno[3,2-d]pyridazine 14

### 3-Formylthiophene-2-carboxylic acid (v)

The compound (v) was prepared from thiophene-2-carboxylic acid by the procedure used for compound (ii) in example 12. ¹H NMR (300 MHz, CDCl₃) δ 10.48 (s, 1H), 7.64 - 7.59 (m, 2H). HRMS (ESI): cal. mass C₆H₄O₃S [M+1]⁺ 156.9954, found [M+1]⁺ 156.9948, cal. mass C₆H₄O₃SNa [M+Na]⁺ 178.9773, found [M+Na]⁺ 178.9770.

### Thieno[2,3-d]pyridazin-7(6H)-one (vi)

Compound (vi) was prepared from compound (v) analogous to the compound (iii) in example 12. ¹H NMR (500 MHz, DMSO) δ 12.93 (s, 1H), 8.44 (s, 1H), 8.19 (d, J= 5.1 Hz, 1H), 7.58 (d, J= 5.1 Hz, 1H). ¹³C NMR (126 MHz, DMSO) δ 158.19, 139.97, 136.24, 135.83, 134.55, 124.82. HRMS (ESI): cal. mass C₆H₄N₂OS [M+1]⁺ 153.0117, found [M+1]⁺ 153.0118, cal. mass C₆H₄N₂OSNa [M+Na]⁺ 174.9937, found [M+Na]⁺ 174.9942.

### 7-Chlorothieno[2,3-d]pyridazine (vii)

Compound (vii) was prepared from compound (vi) according to the compound (iv) in example 12. ¹H NMR (600 MHz, CDCl₃) δ 9.49 (s, 1H), 7.94 (d, J= 5.3 Hz, 1H), 7.58 (d, *J* = 5.3 Hz, 1H). ¹³C NMR (126 MHz, CDCl₃) δ 150.89, 146.07, 139.51, 138.28, 134.53, 123.26. HRMS (ESI): cal. mass C₆H₃ClN₂S [M+1]⁺ 170.9778, found [M+1]⁺ 170.9781, cal. mass C₆H₃ClN₂SNa [M+Na]⁺ 192.9598, found [M+Na]⁺ 192.9596.

### 3-MethylthTetrazolo[1,5-b]thieno[3,2-d]pyridazine 14

The compound **14** was prepared from compound (vii) according to compound **12.** ¹H NMR (600 MHz, CDCl₃) δ 8.69 (s, 1H), 7.73 (d, J= 5.2 Hz, 1H), 7.55 (d, J= 5.2 Hz, 1H), 2.85 (s, 3H). ¹³C NMR (126 MHz, CDCl₃) δ 147.76, 142.17, 141.23, 132.51, 130.76, 129.98, 124.00, 10.31. HRMS (ESI): cal. mass C₈H₆N₄S [M+1]⁺ 191.0386, found [M+1]⁺ 191.0388, cal. mass C₈H₆N₄SNa [M+Na]⁺ 213.0205, found [M+Na]⁺ 213.0207.

### Example 15

### Tetrazolo[1,5-b]thieno[3,2-d]pyridazine 15

Compound **15** was prepared from compound (vii) as described for compound **13.** ¹H NMR (500 MHz, DMSO) δ 9.43 (s, 1H), 8.43 (d, J= 5.1 Hz, 1H), 7.99 (d, J= 5.1 Hz, 1H). ¹³C NMR (126 MHz, DMSO) δ 144.02, 141.33, 134.94, 134.78, 130.73, 125.22. HRMS (ESI): cal. mass C₆H₃N₅S [M+1]⁺ 178.0182, found [M+1]⁺ 178.0183, cal. mass C₆H₃N₅SNa [M+Na]⁺ 200.0001, found [M+Na]⁺ 200.0003.

### Example 16

### 3-Methylthieno[3.4-d][1,2.4]triazolo[4,3-b]pyridazine 16

### Dimethyl thiophene-3,4-dicarboxylate (viii)

A round-bottomed flask was equipped with a stir bar and reflux condenser. The flask was charged with the commercially available thiophene-3,4-dicarboxylic acid (2.5 g, 14.52 mmol, 1.0 equiv.) and dissolved in MeOH (65 mL) with a catalytic amount of H₂SO₄ (~2.5 mL). The reaction was heated to reflux and stirred for 24 h. The reaction was cooled to rt and concentrated rotary evaporation. The residue was purified by silica gel chromatography column and was eluted DCM to yield dimethyl thiophene-3,4-dicarboxylate (viii) as a solid (2.42 g, 83%). ¹H NMR (400 MHz, CDCl₃) δ 7.85 (s, 2H), 3.88 (s, 6H). ¹³C NMR (101 MHz, CDCl₃) δ 163.62, 133.37, 131.97, 52.49. HRMS (ESI): cal. mass C₈H₈O₄S [M+1]⁺ 201.0216, found [M+1]⁺ 201.0216, cal. mass C₈H₈O₄SNa [M+Na]⁺ 223.0036, found [M+Na]⁺ 223.0040.

### 2,3-Dihydrothieno[3,4-d]pyridazine-1,4-dione (ix)

Compound (viii) (2.0 g, 9.99 mmol, 1.0 equiv.) was charged in a round bottomed flask in ethanol (15 mL) and allowed to react with hydrazine (55%) (1.35 g, 26.97 mmol, 2.7 equiv.). The reaction mixture was refluxed for 4 h. After the reaction was completed, the reaction mixture was cooled to room temperature and concentrated under vacuum. The crude solid compound was dissolved in a minimum amount of water containing 1% NH₄OH by volume and precipitated by adding concentrated HCl to the solution. Finally, the obtained solid compound was washed with cold water, MeOH and then dried at room temperature to give (ix) as a solid (1.3 g, 78%). ¹H NMR (400 MHz, DMSO) δ 11.20 (broad singlet, 2H), 8.36 (s, 2H). ¹³C NMR (101 MHz, DMSO) δ 153.12, 130.42, 128.41. HRMS (ESI): cal. mass C₆H₄N₂O₂S [M+1]⁺ 169.0066, found [M+1]⁺ 169.0069, cal. mass C₆H₄N₂O₂SNa [M+Na]⁺ 190.9886, found [M+Na]⁺ 190.9891.

### 1,4-Dichlorothieno[3,4-d]pyridazine (x)

A round-bottomed flask was equipped with a stir bar and reflux condenser. To the flask was added the product (ix) (1.3 g, 7.73 mmol, 1.0 equiv.), phosphorus oxychloride (4 mL), and pyridine (1mL) and refluxed for 16 h under argon. The reaction was cooled to r.t. and poured over ice. The mixture was separated and the aqueous layer was extracted with chloroform (4 × 75 mL). The organic layers were combined, dried (Na₂SO₄) and concentrated by rotary evaporation. The residue was purified by silica gel chromatography column and was eluted DCM to yield (x) as a yellow solid (0.72 g, 46%). ¹H NMR (400 MHz, CDCl₃) δ 8.31 (s, 2H). ¹³C NMR (126 MHz, CDCl₃) δ 150.37, 131.24, 126.40. HRMS (ESI): cal. mass C₆H₂N₂SCl₂ [M+1]⁺ 204.9389, found [M+1]⁺ 204.9389, cal. mass C₆H₂N₂SCl₂Na [M+Na]⁺ 226.9208, found [M+Na]⁺ 226.9209.

### 6-Chloro-3-methylthieno[3,4-d][1,2,4]triazolo[4,3-b]pyridazine (xi)

Compound (x) (0.204 g, 1.0 mmol, 1.0 equiv.) and acetic acid hydrazide (0.222 g, 3.0 mmol, 2.0 equiv.) were refluxed in dioxane (3 mL) for 16 h. The reaction mixture was extracted with Chloroform (4 × 30 mL). Organic phase was dried with Na₂SO₄ and evaporated. The crude product was purified by column chromatography using dichloromethane give an amorphous solid (0.192 g, 86%). ¹H NMR (600 MHz, CDCl₃) δ 8.42 (d, J= 3.0 Hz, 2H), 8.28 (d, J= 3.1 Hz, 2H), 2.75 (s, 6H). ¹³C NMR (126 MHz, CDCl₃) δ 147.78, 145.11, 141.19, 129.60, 127.87, 124.31, 122.63, 10.20. HRMS (ESI): cal. mass C₈H₅N₄SCl [M+1]⁺ 224.9996, found [M+1]⁺ 224.9997, cal. mass C₈H₅N₄SClNa [M+Na]⁺ 246.9816, found [M+Na]⁺ 246.9822.

### 3-Methylthieno[3,4-d][1,2,4]triazolo[4,3-b]pyridazine 16

6-Chloro-3-methylthieno[3,4-d][1,2,4]triazolo[4,3-b]pyridazine (40 mg, 0.17 mmol, 1.0 equiv.) was dissolved in ethanol (3 mL) and hydrazine (55%) (17.8 mg, 0.35 mmol, 2.0 equiv.) was added slowly and the reaction mixture was refluxed for 2 h to give precipitation. The product was collected by suction filtration and dried. Then, a solution of 0.15 M TMSOK (22 mg, 0.17 mmol, 1.0 equiv.) in water (1.6 mL) was added, slurry was stirred for 48 h at room temperature. The resulting mixture was extracted with chloroform (4 × 20 mL). Organic phase was dried with Na₂SO₄ and evaporated to give amorphous solid **16** (12 mg, 35%). ¹H NMR (600 MHz, CDCl₃) δ 8.49 (d, J= 0.9 Hz, 1H), 8.39 (dd, *J* = 2.9, 0.9 Hz, 1H), 8.15 (d, J= 2.9 Hz, 1H), 2.76 (s, 3H). ¹³C NMR (126 MHz, CDCl₃) δ 148.18, 142.27, 141.73, 128.44, 128.31, 124.58, 121.39, 10.26. HRMS (ESI): cal. mass C₈H₆N₄S [M+1]⁺ 191.0386, found [M+1]⁺ 191.0387, cal. mass C₈H₆N₄SNa [M+Na]⁺ 213.0205, found [M+Na]⁺ 213.0199.

### Example 17

### Tetrazolo[1,5-b]thieno[3,2-d]pyridazine 17

### 6-Chlorotetrazolo[1,5-b]thieno[3,4-d]pyridazine (xi)

1,4-Dichlorothieno[3,4-d]pyridazine (x) (0.100 g, 0.49 mmol, 1.0 equiv.) was dissolved methanol (8 mL) and the hydrazine hydrate (55%) (0.049 g, 0.98 mmol, 2.0 equiv.) was added slowly and the reaction mixture was refluxed for 1 h. After the reaction was completed, the reaction mixture was cooled to room temperature and concentrated under vacuum to give crude yellow precipitation. Which was dissolved in acetic acid (5 mL, 2N) and under stirring the ice-cold solution was treated with a cold aqueous solution of NaNO₂ (50 mg, 0.73 mmol, 1.5 equiv. in 2 mL water) dropwise. The reaction mixture was stirred at room temperature for 1 h. The compound was extracted with chloroform (3 × 25 mL). Organic phase was dried with Na₂SO₄ and evaporated to give an amorphous solid (xi)(48 mg, 46%). ¹H NMR (500 MHz, DMSO) δ 8.99 (d, J= 2.8 Hz, 1H), 8.80 (d, J= 2.8 Hz, 1H). ¹³C NMR (126 MHz, DMSO) δ 150.35, 140.43, 130.79, 126.37, 124.04, 121.94.

### Tetrazolo[1,5-b]thieno[3,4-d]pyridazine 17

6-Chlorotetrazolo[1,5-b]thieno[3,4-d]pyridazine (40 mg, 0.18 mmol, 1.0 equiv.) was dissolved in ethanol (3 mL) and the hydrazine hydride (55%) (18.9 mg, 0.37 mmol, 2.0 equiv.) was added slowly and the reaction mixture was refluxed for 2 h to give precipitation. The product was collected by suction filtration and dried. Then, a solution of 0.15 M TMSOK (24 mg, 0.18 mmol, 1.0 equiv.) in water (1.7 mL) was added, slurry was stirred for 48 h at room temperature. The resulting mixture was extracted with Chloroform (4 × 20 mL). Organic phase was dried with Na₂SO₄ and evaporated to give amorphous solid **17** (8 mg, 23%). ¹H NMR (600 MHz, CDCl₃) δ 9.34 (s, 1H), 8.48 (d, J= 3.2 Hz, 1H), 7.96 (d, J= 3.2 Hz, 1H). ¹³C NMR (126 MHz, CDCl₃) δ 157.22, 135.32, 132.41, 131.03, 130.35, 126.09. HRMS (ESI): cal. mass C₆H₃N₅S [M+1]⁺ 178.0182, found [M+1]⁺ 178.0192.

### Example 18

### 10-Chloro-3-methyl-[1,2,4]triazolo[3,4-a]phthalazine 18

### 5-Chloro-2,3-dihydrophthalazine-1,4-dione (xii)

4-Chloroisobenzofuran-1,3-dione (10 g, 54.76 mmol, 1.0 equiv.) was dissolved in 10% HCl (50 mL), and hydrazine hydrate (55%) (2.63 g, 82.14 mmol, 1.5 equiv.) was added and the mixture was refluxed with stirring for 16 h. The product was collected by suction filtration, washed with water and dried, to yield an amorphous solid (xii)(10.1 g, 93%). ¹H NMR (300 MHz, DMSO) δ 11.60 (s, 2H), 8.00 (d, J= 7.6 Hz, 1H), 7.84 (m, 2H). HRMS (ESI): cal. mass C₈H₅ClN₂O₂ [M+1]⁺ 197.0112, found [M+1]⁺ 197.0110, cal. mass C₈H₅ClN₂O₂ [M+Na]⁺ 218.9932, found [M+Na]⁺ 218.9938.

### 1,4,5-Trichlorophthalazine (xiii)

A round-bottomed flask was equipped with a stir bar and reflux condenser. To the flask was added the 5-chloro-2,3-dihydrophthalazine-1,4-dione (10 g, 50.86 mmol, 1.0 equiv.), phosphorus oxychloride (23.65 mL, 508.64 mmol, 5.0 equiv.) and refluxed for 24 h. The reaction was cooled to rt and poured over ice. The mixture was separated and the aqueous layer was extracted with chloroform (4 × 60 mL). The organic layers were combined, dried (Na₂SO₄) and concentrated by rotary evaporation. The crude product was purified by column chromatography using dichloromethane give amorphous solid (xiii)(2.1 g, 26%). ¹H NMR (400 MHz, CDCl₃) δ 8.34 (dd, *J* = 8.3, 1.2 Hz, 1H), 8.11 (dd, *J =* 7.8, 1.2 Hz, 1H), 7.94 (dd, *J* = 8.3, 7.8 Hz, 1H). ¹³C NMR (126 MHz, CDCl₃) δ 155.28, 152.45, 137.79, 134.17, 132.06, 129.45, 125.83, 124.59. HRMS (ESI): cal. mass C₈H₃Cl₃N₂ [M+1]⁺ 232.9435, found [M+1]⁺ 232.9441, cal. mass C₈H₃Cl₃N₂ [M+Na]⁺ 254.9254, found [M+Na]⁺ 254.9263.

### 6,10-Dichloro-3-methyl-[1,2,4]triazolo[3,4-a]phthalazine (xiv) (major isomer) and 6,7-dichloro-3-methyl-[1,2,4]triazolo[3,4-a]phthalazine (xv) (minor isomer)

1,4,5-Trichlorophthalazine (xiii) (500 mg, 2.15 mmol, 1.0 equiv.) and acetic acid hydrazide (318 mg, 4.31 mmol, 2.0 equiv.) were refluxed in dioxane (6 mL) for 4 h. The reaction mixture was extracted with dichloromethane (4 × 40 mL). Organic phase was dried with Na₂SO₄ and concentrated by rotary evaporation to give a solid (xiv)/(xv) (421 mg, 77%). ¹H NMR (300 MHz, CDCl₃) δ 8.67 (dd, J= 7.4, 2.0 Hz, 0.17H), 8.23 (dd, *J =* 8.1, 1.1 Hz, 1H), 8.03 (dd, *J* = 8.0, 1.2 Hz, 1H), 7.88 - 7.80 (m, 0.36H), 7.77 (t, J= 8.1 Hz, 1H), 2.83 (s, 3H), 2.81 (s, 0.60H). HRMS (ESI): cal. Mass C₁₀H₆Cl₂N₄ [M+1]⁺ 253.0042, found [M+1]⁺ 253.0042, cal. Mass C₁₀H₆Cl₂N₄ [M+Na]⁺ 274.9862, found [M+Na]⁺ 274.9868.

Mixture of isomers were separated by column chromatography. First isomer: 6,10-dichloro-3-methyl-[1,2,4]triazolo[3,4-a]phthalazine (xiv) (major isomer) ¹H NMR (300 MHz, CDCl₃) δ 8.23 (dd, *J* = 8.1, 1.3 Hz, 1H), 8.04 (dd, *J=* 8.1, 1.3 Hz, 1H), 7.77 (t, *J =* 8.1Hz, 1H), 2.83 (s, 3H). second isomer: 6,7-dichloro-3-methyl-[1,2,4]triazolo[3,4-a]phthalazine (xv) (minor isomer) ¹H NMR (300 MHz, CDCl₃) δ 8.68 (dd, *J=* 7.3, 1.9 Hz, 1H), 7.91 - 7.80 (m, 2H), 2.81 (s, 3H).

### 10-Chloro-3-methyl-[1,2,4]triazolo[3,4-a]phthalazine 18

Compound (xiv) (major isomer) (250 mg, 0.98 mmol, 1.0 equiv.) was dissolved in ethanol (5 mL) and hydrazine (55%) (63.24 mg, 1.97 mmol, 2.0 equiv.) was added slowly and the reaction mixture was refluxed for 3 h to give precipitation. The product was collected by suction filtration and dried. Then, a solution of 0.15 M TMSOK (126 mg, 0.98 mmol, 1.0 equiv.) in water was added, slurry was stirred for 48 h at room temperature. The resulting mixture was extracted with chloroform (4 × 30 mL). Organic phase was dried with Na₂SO₄ and evaporated. The crude product was purified by column chromatography using dichloromethane give amorphous solid **18** (45 mg, 21%). ¹H NMR (300 MHz, CDCl₃) δ 8.59 (s, 1H), 7.98 (dd, *J=* 7.9, 1.2 Hz, 1H), 7.84 (dd, *J=* 7.8, 1.2 Hz, 1H), 7.71 (t, *J* = 7.9 Hz, 1H), 2.84 (s, 3H). HRMS (ESI): cal. mass C₁₀H₇ClN₄ [M+1]⁺ 219.0432, found [M+1]⁺ 219.0435, cal. mass C₁₀H₇ClN₄ [M+Na]⁺ 241.0251, found [M+Na]⁺ 241.0259.

### Example 19

### 10-chlorotetrazolo[5,1-a]phthalazine 19

### 6,10-dichlorotetrazolo[5,1-a]phthalazine (xvi) and 6,7-dichlorotetrazolo[5,1-a]phtha lazine (xvii)

1,4,5-trichlorophthalazine (xxiii) (800 mg, 3.44 mmol, 1.0 equiv.) was dissolved ethanol (10 mL) and hydrazine (55%) (221 mg, 6.89 mmol, 2.0 equiv.) was added slowly and the reaction mixture was refluxed for 1 h. After the reaction was completed, the reaction mixture was cooled to room temperature and concentrated under vacuum to give crude yellow precipitation. Which was dissolved in acetic acid (15 mL, 2N) and under stirring the ice-cold solution was treated with a cold aqueous solution of NaNO₂ (356 mg, 5.17 mmol, 1.5 equiv. in 2 mL water) dropwise. The reaction mixture was stirred at room temperature for 1 h. The compound was extracted with chloroform (3 × 35 mL). Organic phase was dried with Na₂SO₄ and evaporated to give an amorphous solid (452 mg, 55%). Mixture of isomers were separated by column chromatography using DCM/MeOH 50:1. First isomer 6,10-dichlorotetrazolo[5,1-a]phthalazine (xvi) ¹H NMR (300 MHz, CDCl₃) δ 8.43 (dd, J= 8.1, 1.1 Hz, 1H), 8.21 (dd, J= 8.1, 1.1 Hz, 1H), 8.01 (t, J= 8.1 Hz, 1H). HRMS (ESI): cal. mass C₈H₃Cl₂N₅ [M+1]⁺ 239.9838, found [M+1]⁺ 239.9842, cal. mass C₈H₃Cl₂N₅ [M+Na]⁺ 261.9658, found [M+Na]⁺ 261.9664. Second isomer 6,7-dichlorotetrazolo[5,1-a]phthalazine (xvii) ¹H NMR (300 MHz, CDCl₃) δ 8.20 (dd, *J* = 8.1, 1.1 Hz, 1H), 8.13 (dd, J= 8.1, 1.1 Hz, 1H), 7.89 (t, *J* = 8.1 Hz, 1H).

### 10-Chlorotetrazolo[5,1-a]phthalazine 19

This was prepared from 6,10-dichlorotetrazolo[5,1-a]phthalazine (xvi) and 6,7-dichlorotetrazolo[5,1-a]phthalazine (xvii) according to the example 18. Yield 22%. Mixture of isomers were separated by column chromatography using DCM. First isomer 10-chlorotetrazolo[5,1-a]phthalazine **19** ¹H NMR (600 MHz, CDCl₃) δ 8.95 (s, 1H), 8.16 (dd, *J=* 7.9, 1.1 Hz, 1H), 8.07 (dd, J= 7.9, 1.1 Hz, 1H), 7.95 (t, J= 7.9 Hz, 1H). ¹³C NMR (126 MHz, CDCl₃) δ 148.91, 140.92, 136.18, 132.89, 132.77, 127.09, 126.44, 121.19.

### Example 20

### 7-Chlorotetrazolo[5,1-a]phthalazine 20

This was prepared from 6,10-dichlorotetrazolo[5,1-a]phthalazine (xvi) and 6,7-dichlorotetrazolo[5,1-a]phthalazine (xvii) according to the example 18. Yield 22%. Mixture of isomers were separated by column chromatography using DCM. Second isomer 7-chlorotetrazolo[5,1-a]phthalazine **20.** ¹H NMR (600 MHz, DMSO) δ 9.56 (s, 1H), 8.64 (d, J= 7.8 Hz, 1H), 8.24 (dd, J= 7.9, 1.1 Hz, 1H), 8.19 (t, J= 7.9 Hz, 1H). ¹³C NMR (126 MHz, DMSO) δ 147.01, 142.05, 136.46, 133.70, 133.43, 124.28, 123.42, 122.98. HRMS (ESI): cal. mass C₈H₄N₅Cl [M+1]⁺ 206.0228, [M+2]⁺ 208.0199 found [M+1]⁺ 206.0234, [M+2]⁺ 208.0230 cal. mass C₈H₄N₅Cl Na [M+1]⁺ 228.0047, [M+2]⁺ 230.0018 found [M+1]⁺ 228.0045, [M+2]⁺ 230.0018.

### Example 21

### 9-chloro-3-methyl-[1,2,4]triazolo[3,4-a]phthalazine 21

### 6-chloro-2,3-dihydrophthalazine-1,4-dione (xviii)

This was prepared from 5-chloroisobenzofuran-1,3-dione according to the compound (xii) in example 18. Yield 98%.¹H NMR (300 MHz, DMSO) δ 8.07 (d, J= 8.6 Hz, 1H), 8.00 (d, J= 2.1 Hz, 1H), 7.90 (dd, J= 8.5, 2.2 Hz, 1H). ¹³C NMR (75 MHz, DMSO) δ 138.07, 136.13, 133.32, 131.08, 130.99, 128.40, 128.16, 124.90.

### 1,4,6-trichlorophthalazine (xix)

This was prepared from (xviii) according to the procedure given for compound (xiii) in example 18. Yield 51%. ¹H NMR (300 MHz, CDCl₃) δ 8.31 - 8.26 (m, 2H), 8.01 (ddd, J= 8.9, 2.0, 0.9 Hz, 1H). ¹³C NMR (75 MHz, CDCl₃) δ 154.80, 154.07, 141.39, 135.55, 128.25, 127.94, 125.71, 125.28.

### 6,9-dichloro-3-methyl-[1,2,4]triazolo[3,4-a]phthalazine (xx) and 6,8-dichloro-3-methyl-[1,2,4]triazolo[3,4-a]phthalazine (xxi)

This was prepared from (xix) according to the procedure given for compound (xiv) in example 18. Yield 91%.¹H NMR (300 MHz, CDCl₃) δ 8.64 (d, *J* = 2.1 Hz, 1H), 8.62 (d, J= 8.6 Hz, 1H), 8.23 (d, *J* = 2.0 Hz, 1H), 8.19 (d, *J* = 8.8 Hz, 1H), 7.97 - 7.91 (m, 1H), 7.82 - 7.76 (m, 1H), 2.81 (s, 6H). ¹³C NMR (75 MHz, CDCl₃) δ 149.30, 148.69, 148.53, 148.03, 141.85, 137.78, 135.43, 131.88, 129.21, 127.18, 125.33, 125.29, 123.39, 123.31, 122.52, 120.42, 9.93 (2 × CH₃).

### 9-Chloro-3-methyl-[1,2,4]triazolo[3,4-a]phthalazine 21

This was prepared from (xx/xxi) according to the procedure given in example 18, yield 26%. The mixture of isomers were separated by column chromatography (DCM/MeOH 50:1). 9-chloro-3-methyl-[1,2,4]triazolo[3,4-a]phthalazine **21** (first isomer). ¹H NMR (600 MHz, CDCl₃) δ 8.64 (d, *J* = 2.0 Hz, 1H), 8.60 (s, 1H), 7.87 (d, *J=* 8.5 Hz, 1H), 7.75 (dd, *J =* 8.4, 2.0 Hz, 1H), 2.83 (s, 3H). ¹³C NMR (126 MHz, CDCl₃) δ 148.57, 146.73, 141.77, 140.76, 131.52, 129.63, 124.86, 123.17, 121.27, 10.03.

### Example 22

### 8-Chloro-3-methyl-[1,2,4]triazolo[3,4-a]phthalazine 22

This was prepared from (xx/xxi) according to the procedure given in example 18. Mixture of isomers were separated by column chromatography (DCM/MeOH 50:1). 8-chloro-3-methyl-[1,2,4]triazolo[3,4-a]phthalazine **22** (second isomer). ¹H NMR (600 MHz, CDCl₃) δ 8.68 (d, J= 8.4 Hz, 1H), 8.60 (s, 1H), 7.95 - 7.91 (m, 2H), 2.85 (s, 3H). ¹³C NMR (126 MHz, CDCl₃) δ 148.47, 146.48, 142.10, 137.20, 134.72, 127.62, 125.18, 124.17, 121.93, 10.05.

### Example 23

### 9-Chlorotetrazolo[5,1-a]phthalazine 23

### 6,9-dichlorotetrazolo[5,1-a]phthalazine (xxii) and 6,8-dichlorotetrazolo[5,1-a]phthalazine (xxiii)

This was prepared from compound (xix) according to the procedure given in example 18. Yield (71%). ¹H NMR (300 MHz, CDCl₃) δ 8.78 - 8.71 (m, 1.4H), 8.46 - 8.37 (m, 1.4H), 8.13 (dt, J= 8.6, 1.7 Hz, 1H), 8.03 (dt, J= 8.9, 1.8 Hz, 0.4H). ¹³C NMR (75 MHz, CDCl₃) δ 151.82, 151.79, 151.11, 143.01, 141.55, 140.36, 136.39, 134.15, 129.72, 127.74, 126.80, 125.45, 124.90, 123.61, 122.57, 120.84.

### 9-chlorotetrazolo[5,1-a]phthalazine 23

This was prepared from (xxii/xxiii) according to the procedure given in example 19. Yield (21%). Mixture of isomers were separated by column chromatography using DCM. 9-chlorotetrazolo[5,1-a]phthalazine **23** (first isomer). ¹H NMR (600 MHz, CDCl₃) δ 8.94 (d, J= 0.7 Hz, 1H), 8.74 (dd, J= 2.0, 0.6 Hz, 1H), 8.11 (dd, *J=* 8.5, 0.5 Hz, 1H), 7.97 (dd, J= 8.5, 2.0 Hz, 1H). ¹³C NMR (126 MHz, CDCl₃) δ 148.50, 141.94, 141.42, 133.62, 130.11, 124.52, 123.63, 123.14.

### Example 24

### 8-chlorotetrazolor 5, 1-a lphthalazine 24

This was prepared from (xxii/xxiii) according to the procedure given in example 19. Yield (21%). Mixture of isomers were separated by column chromatography using DCM. 8-chlorotetrazolo[5,1-a]phthalazine **24** (second isomer). ¹H NMR (600 MHz, CDCl₃) δ 8.90 (s, 1H), 8.72 (d, J= 8.5 Hz, 1H), 8.14 (d, *J* = 2.0 Hz, 1H), 8.08 (dd, *J* = 8.6, 2.0 Hz, 1H). ¹³C NMR (126 MHz, CDCl₃) δ 148.07, 141.82, 139.45, 135.60, 128.08, 126.39, 125.98, 120.82.

### Example 25

### 8,9-dichloro-3-methyl-[1,2,4]triazolo[3,4-a]phthalazine 25

### 6,7-dichloro-2,3-dihydrophthalazine-1,4-dione (xxiv)

To a solution of 4,5-dichlorophthalic acid (10 g, 42.55 mmol) in AcCl (60 mL) was refluxed for 2 h and then concentrated to afford a light brown solid 5,6-dichloroisobenzofuran-1,3-dione (9.21 g, 99% yield) and the crude product was directly subjected to the next reaction without further purification. ¹H NMR (300 MHz, CDCl₃) δ 8.11 (s, 2H). ¹³C NMR (75 MHz, CDCl₃) δ 160.82, 141.87, 130.42, 127.57.

To a solution of 5,6-dichloroisobenzofuran-1,3-dione (9.2 g, 42.39 mmol) in 10% HCl (60 mL) hydrazine (55%) (2.71 g, 84.79 mmol) was added and the mixture was refluxed and stirred for 24 h. The product was collected by suction filtration, washed with water and dried, to yield as an amorphous solid (xxiv) (9.51 g, 94%).

### 1,4,6,7-tetrachlorophthalazine (xxv)

This was prepared from (xxiv) according to the procedure given for compound (xxiii) in example 18. Yield 29%. ¹H NMR (300 MHz, CDCl₃) δ 8.41 (s, 2H). ¹³C NMR (75 MHz, CDCl₃) δ 153.59, 140.40, 127.53, 126.21.

### 6,8,9-trichloro-3-methyl-[1,2,4]triazolo[3,4-a]phthalazine (xxvi)

This was prepared from (xxv) according to the procedure given in example 18. Yield (74%). ¹H NMR (300 MHz, CDCl₃) δ 8.75 (s, 1H), 8.33 (s, 1H), 2.81 (s, 3H). ¹³C NMR (75 MHz, CDCl₃) δ 148.41, 148.08, 141.53, 140.50, 136.37, 129.19, 125.34, 123.18, 121.32, 9.94.

### 8,9-dichloro-3-methyl-[1,2,4]triazolo[3,4-a]phthalazine_25_

This was prepared from (xxvi) according to the procedure given in example 18. Yield (19%). ¹H NMR (300 MHz, CDCl₃) δ 8.76 (s, 1H), 8.56 (s, 1H), 8.04 (d, J= 1.1 Hz, 1H), 2.84 (d, J= 1.1 Hz, 3H).

### Example 26

### 8,9-dichlorotetrazolo[5,1-a]phthalazine 26

### 6,8,9-trichlorotetrazolo[5,1-a]phthalazine (xxvii)

This was prepared from compound 1,4,6,7-tetrachlorophthalazine (xxv) given in example 25 by the procedure given in example 19. Yield (51%). ¹H NMR (300 MHz, CDCl₃) δ 8.87 (s, 1H), 8.53 (s, 1H). ¹³C NMR (75 MHz, CDCl₃) δ 141.79, 140.80, 140.78, 139.14, 129.66, 126.78, 123.36, 121.40.

### 8,9-dichlorotetrazolo[5,1-a]phthalazine 26

This was prepared from (xxvii) according to the procedure given in example 18. Yield (26%). ¹H NMR (600 MHz, CDCl₃) δ 8.89 (s, 1H), 8.86 (s, 1H), 8.26 (s, 1H). ¹³C NMR (126 MHz, CDCl₃) δ 147.48, 140.98, 140.71, 138.31, 129.96, 126.48, 123.89, 121.52.

### Example 27

### 8,9-dichloro-3-methyl-[1,2,4]triazolo[3,4-a]phthalazine 27

### 5-Fluoro-2,3-dihydrophthalazine-1,4-dione (xxviii)

This was prepared from commercially available 4-fluoroisobenzofuran-1,3-dione according to the compound (xii) in example 18. Yield (95%). ¹H NMR (300 MHz, DMSO) δ 11.58 (s, 2H), 7.95 - 7.81 (m, 2H), 7.73 - 7.58 (m, 1H). ¹⁹F NMR (282 MHz, DMSO) δ -111.42. HRMS (ESI): cal. mass C₈H₅N₂O₂F [M+1]⁺ 181.0408, found [M+1]⁺ 181.0404, cal. mass C₈H₅N₂O₂F [M+Na]⁺ 203.0227, found [M+Na]⁺ 203.0237.

### 1,4-Dichloro-5-fluorophthalazine (xxix)

This was prepared from (xxviii) according to the procedure given for compound (xiii) in example 18. Yield (38%). ¹H NMR (300 MHz, CDCl₃) δ 8.18 (dd, *J* = 8.4, 1.0 Hz, 1H), 8.11 - 7.97 (m, 1H), 7.80 - 7.67 (m, 1H). HRMS (ESI): cal. mass C₈H₃N₂Cl₂F [M+1]⁺ 216.9730, found [M+1]⁺ 216.9724, cal. mass C₈H₃N₂Cl₂F [M+Na]⁺ 238.9550, found [M+Na]⁺ 240.9520.

### 6-Chloro-10-fluoro-3-methyl-[1,2,4]triazolo[3,4-a]phthalazine (xxx) and 6-chloro-7-fluoro-3-methyl-[1,2,4]triazolo[3,4-a]phthalazine (xxxi)

This was prepared from (xxix) according to the procedure given in example 18. Yield (82%). ¹H NMR (300 MHz, CDCl₃) δ 8.52 - 8.47 (m, 0.5H), 8.10 (dd, *J=* 8.1, 1.1 Hz, 1H), 7.95 (td, *J=* 8.1, 4.6 Hz, 0.5H), 7.84 (td, *J=* 8.2, 5.0 Hz, 1H), 7.75 (td, *J=* 8.7, 8.1, 1.1 Hz, 1H), 7.57 - 7.46 (m, 0.5H), 2.82 (s, 3H), 2.81 (s, 1.5H).

### 10-fluoro-3-methyl-[1,2,4]triazolo[3,4-a]phthalazine 27 and 7-fluoro-3-methyl-[1,2,4]triazolo[3,4-a]phthalazine 27b

This was prepared from (xxx/xxxi) according to the procedure given in example 18. Yield (31%). ¹H NMR (300 MHz, CDCl₃) δ 8.91 (s, 1H), 8.63 (d, *J =* 2.1 Hz, 1H), 8.44 (d, *J* = 8.0 Hz, 1H), 7.91 (td, J= 8.1, 5.3 Hz, 1H), 7.81 - 7.65 (m, 3H), 7.51 - 7.43 (m, 1H), 2.84 (s, 6H). ¹⁹F NMR (282 MHz, CDCl₃) δ -107.77, -118.06.

10-Fluoro-3-methyl-[1,2,4]triazolo[3,4-a]phthalazine **27** was isolated from the above mixture by crystallization from hexane. ¹H NMR (600 MHz, DMSO) δ 9.02 (s, 1H), 8.11 - 8.07 (m, 1H), 7.83 - 7.78 (m, 2H), 2.62 (s, 3H). ¹³C NMR (126 MHz, DMSO) δ 158.57, 156.53, 152.55, 146.03, 138.39, 131.23, 119.78, 119.31, 112.62, 9.40.

### Example 28

### 7-Fluorotetrazolo[5,1-a]phthalazine 28

### 6-Chloro-10-fluorotetrazolo[5,1-a]phthalazine (xxxii) and 6-chloro-7-fluorotetrazolo[5,1-a]phthalazine (xxxiii)

This was prepared from compound (xxix) according to the procedure given in example 19. Yield (69%). ¹H NMR (300 MHz, CDCl₃) δ 8.62 (d, J= 8.0 Hz, 0.5 H), 8.31 (d, *J* = 8.0 Hz, 1H), 8.19 - 8.07 (m, 1.5H), 7.92 (td, J= 8.5, 0.9 Hz, 1H), 7.75 (ddd, J= 11.6, 8.3, 1.1 Hz, 0.5H).

### 7-Fluorotetrazolor5,1-a]phthalazine 28

This was prepared from compound (xxxii/xxxiii) according to the procedure given in example 19. Yield (37%). ¹H NMR (300 MHz, CDCl₃) δ 9.23 (s, 0.3H), 8.98 (s, 1H), 8.56 (d, J= 8.1 Hz, 0.3H), 8.15 - 8.07 (m, 0 .3H), 8.05 - 7.94 (m, 2H), 7.86 (td, *J* = 8.3, 7.4, 1.6 Hz, 1H), 7.69 (t, *J* = 8.9 Hz, 0.3H). ¹⁹F NMR (282 MHz, CDCl₃) δ - 106.33, -115.77. Mixture of isomers were separated by column chromatography. 7-fluorotetrazolo[5,1-a]phthalazine **28** (first isomer). ¹H NMR (400 MHz, CDCl₃) δ 9.24 (d, J= 0.8 Hz, 1H), 8.56 (d, J= 8.0 Hz, 1H), 8.11 (td, *J* = 8.1, 5.2 Hz, 1H), 7.69 (ddd, J= 9.3, 8.3, 0.9 Hz, 1H).

### Example 29

### 10-Fluorotetrazolo[5,1-a]phthalazine 29_

This was prepared from compound (xxxii/xxxiii) according to the procedure given in example 19. Yield (37%). ¹H NMR (300 MHz, CDCl₃) δ 9.23 (s, 0.3H), 8.98 (s, 1H), 8.56 (d, J= 8.1 Hz, 0.3H), 8.15 - 8.07 (m, 0 .3H), 8.05 - 7.94 (m, 2H), 7.86 (td, *J* = 8.3, 7.4, 1.6 Hz, 1H), 7.69 (t, J= 8.9 Hz, 0.3H). ¹⁹F NMR (282 MHz, CDCl₃) δ - 106.33, -115.77. Mixture of isomers were separated by column chromatography. 10-fluorotetrazolo[5,1-a]phthalazine **29** (second isomer). ¹H NMR (400 MHz, CDCl₃) δ 8.98 (d, J= 2.0 Hz, 1H), 8.08 - 7.96 (m, 2H), 7.86 (ddd, J= 9.2, 7.8, 1.3 Hz, 1H).

### Example 30

### 9-Fluoro-3-methyl-[1,2,4]triazolo[3,4-a]phthalazine 30_

### 6-fluoro-2,3-dihydrophthalazine-1,4-dione (xxxiv)

This was prepared from commercially available 5-fluoroisobenzofuran-1,3-dione according to the compound (xxii) in example 18. Yield (96%). ¹H NMR (300 MHz, DMSO) δ 11.67 (s, 2H), 8.15 (dd, J = 9.6, 5.3 Hz, 1H), 8.17 - 7.69 (m, 2H). ¹⁹F NMR (282 MHz, DMSO) δ -105.04.

### 1,4-dichloro-6-fluorophthalazine (xxxv)

This was prepared from (xxxiv) according to the procedure given compound (xii) in example 18. Yield (53%). ¹H NMR (300 MHz, CDCl₃) δ 8.39 (dd, *J=* 9.1, 5.0 Hz, 1H), 7.94 (dd, *J* = 8.1, 2.5 Hz, 1H), 7.80 (ddd, *J=* 9.1, 8.1, 2.5 Hz, 1H). HRMS (ESI): cal. mass C₈H₃N₂Cl₂F [M+1]⁺ 216.9730, found [M+1]⁺ 216.9742, cal. mass C₈H₃N₂Cl₂F [M+Na]⁺ 238.9550, found [M+Na]⁺ 240.9560.

### 6-Chloro-9-fluoro-3-methyl-[1,2,4]triazolo[3,4-a]phthalazine (xxxvi) and 6-chloro-8-fluoro-3-methyl-[1,2,4]triazolo[3,4-a]phthalazine (xxxvii)

This was prepared from 1,4-dichloro-6-fluorophthalazine (xxxv) according to the procedure given in example 18. Yield (80%). ¹H NMR (300 MHz, CDCl₃) δ 8.68 (dd, *J* = 8.8, 5.1 Hz, 1H), 8.33 - 8.24 (m, 2H), 7.91 (dd, *J=* 8.8, 2.5 Hz, 1H), 7.72 (td, *J* = 8.4, 2.5 Hz, 1H), 7.54 (ddd, J= 9.1, 8.1, 2.6 Hz, 1H), 2.81 (s, 3H), 2.80 (s, 3H). HRMS (ESI): cal. mass C₁₀H₆N₄ClF [M+1]⁺ 237.0338, found [M+1]⁺ 237.0346, cal. mass C₁₀H₆N₄ClF [M+Na]⁺ 259.0157, found [M+Na]⁺ 259.0167.

### 9-fluoro-3-methyl-[1,2,4]triazolo[3,4-a]phthalazine 30

This was prepared from (xxxvi/xxxvii) according to the procedure given in example 18. Yield (24%). Mixture of isomers were separated by column chromatography using DCM. HRMS (ESI): cal. mass C₁₀H₇N₄F [M+1]⁺ 203.0728, found [M+1]⁺ 203.0722, cal. mass C₁₀H₇N₄F [M+Na]⁺ 225.0547, found [M+Na]⁺ 225.0542. 9-fluoro-3-methyl-[1,2,4]triazolo[3,4-a]phthalazine **30** (first isomer) ¹H NMR (500 MHz, CDCl₃) δ 8.61 (s, 1H), 8.32 (dd, *J=* 8.5, 2.5 Hz, 1H), 7.98 (dd, *J=* 8.7, 5.0 Hz, 1H), 7.52 (td, J= 8.5, 2.5 Hz, 1H), 2.85 (s, 3H

### Example 31

### 8-fluoro-3-methyl-[1,2,4]triazolo[3,4-a]phthalazine 31

This was prepared from (xxxvi/xxxvii) according to the procedure given in example 18. Yield (24%). Mixture of isomers were separated by column chromatography using DCM. HRMS (ESI): cal. mass C₁₀H₇N₄F [M+1]⁺ 203.0728, found [M+1]⁺ 203.0722, cal. mass C₁₀H₇N₄F [M+Na]⁺ 225.0547, found [M+Na]⁺ 225.0542. 8-fluoro-3-methyl-[1,2,4]triazolo[3,4-a]phthalazine **31** (second isomer) ¹H NMR (500 MHz, CDCl₃) δ 8.68 (dd, *J=* 8.7, 5.0 Hz, 1H), 8.58 (s, 1H), 7.69 (td, *J* = 8.5, 2.5 Hz, 1H), 7.59 (dd, J= 8.1, 2.5 Hz, 1H), 2.82 (s, 3H).

### Example 32

### 9-Fluorotetrazolo[5,1-a]phthalazine 32

### 6-Chloro-9-fluorotetrazolo[5,1-a]phthalazine (xxxviii) and 6-chloro-8-fluorotetrazolo[5,1-a]phthalazine (xxxix)

This was prepared from compound 1,4-dichloro-6-fluorophthalazine (xxv) according to the procedure given in example 18. Yield (77%). ¹H NMR (300 MHz, CDCl₃) δ 8.82 (dd, J= 8.8, 5.0 Hz, 1H), 8.52 (dd, J= 9.1, 4.9 Hz, 1H), 8.41 (dd, J= 7.6, 2.6 Hz, 1H), 8.11 (dd, *J =* 8.5, 2.5 Hz, 1H), 7.91 (td, *J=* 8.4, 2.5 Hz, 1H), 7.80 (td, *J* = 8.5, 2.5 Hz, 1H). HRMS (ESI): cal. mass C₈H₃N₅FCl [M+1]⁺ 224.0134, found [M+1]⁺ 224.0145, cal. mass C₈H₃N₅FCl [M+Na]⁺ 245.9953, found [M+Na]⁺ 245.9961.

### 9-Fluorotetrazolo[5,1-a]phthalazine 32

This was prepared from example (xxxviii/xxxix) according to the procedure given in example 18. Yield (25%). ¹H NMR (300 MHz, CDCl₃) δ 8.94 (s, 0.3H), 8.93 (s, 1H), 8.80 (dd, J= 8.8, 4.9 Hz, 1H), 8.40 (dd, *J* = 7.8, 2.5 Hz, 0.3H), 8.21 (dd, J= 8.8, 4.9 Hz, 0.3H), 7.89 - 7.80 (m, 2H), 7.74 (td, *J* = 8.5, 2.4 Hz, 0.3H). HRMS (ESI): cal. mass C₈H₄N₅F [M+1]⁺ 190.0523, found [M+1]⁺ 190.0519, cal. mass C₈H₄N₅F [M+Na]⁺ 212.0343, found [M+Na]⁺ 212.0342. Mixture of isomers were separated by column chromatography using DCM. 9-fluorotetrazolo[5,1-a]phthalazine **32** (first isomer) ¹H NMR (500 MHz, CDCl₃) δ 8.94 (s, 1H), 8.40 (dd, *J* = 7.8, 2.5 Hz, 1H), 8.21 (dd, *J* = 8.8, 4.9 Hz, 1H), 7.74 (ddd, J= 8.8, 8.2, 2.5 Hz, 1H). ¹⁹F NMR (282 MHz, CDCl₃) δ -97.05 (td, J= 8.1, 4.8 Hz).

### Example 33

### 8-fluorotetrazolo[5,1-a]phthalazine 33

This was prepared from example (xxxviii/xxxix) according to the procedure given in example 18. Yield (25%). ¹H NMR (300 MHz, CDCl₃) δ 8.94 (s, 0.3H), 8.93 (s, 1H), 8.80 (dd, J= 8.8, 4.9 Hz, 1H), 8.40 (dd, J= 7.8, 2.5 Hz, 0.3H), 8.21 (dd, J= 8.8, 4.9 Hz, 0.3H), 7.89 - 7.80 (m, 2H), 7.74 (td, *J* = 8.5, 2.4 Hz, 0.3H). HRMS (ESI): cal. mass C₈H₄N₅F [M+1]⁺ 190.0523, found [M+1]⁺ 190.0519, cal. mass C₈H₄N₅F [M+Na]⁺ 212.0343, found [M+Na]⁺ 212.0342. Mixture of isomers were separated by column chromatography using DCM. 8-fluorotetrazolo[5,1-a]phthalazine **33** (second isomer) ¹H NMR (500 MHz, CDCl₃) δ 8.92 (s, 1H), 8.80 (dd, J= 8.8, 4.9 Hz, 1H), 7.90 - 7.79 (m, 2H). ¹⁹F NMR (282 MHz, CDCl₃) δ -102.02 (td, J= 7.9, 4.8 Hz).

### Example 34

### 8,9-dichloro-3-methyl-[1,2,4]triazolo[3,4-a]phthalazine 34

### 6,7-difluoro-2,3-dihydrophthalazine-1,4-dione (xl)

To a solution of 4,5-difluorophthalic acid (5 g, 24.74 mmol) in Ac₂O (30 mL) was refluxed for 2 h and then concentrated to afford a light brown solid 5,6-difluoroisobenzofuran-1,3-dione (4.51 g, 99% yield) and the crude product was directly subjected to the next reaction without further purification. ¹H NMR (300 MHz, CDCl₃) δ 7.83 (t, J = 7.0 Hz, 2H).

To a solution of 5,6-difluoroisobenzofuran-1,3-dione (4.5 g, 24.59 mmol) in ethanol (50 mL) hydrazine (55%) (1.57 g, 49.18 mmol, 2.0 equiv.) was added and the mixture was refluxed and stirred for 24 h. The product was collected by suction filtration, washed with water and dried, to yield as an amorphous solid (xl) (4.62 g, 95%). ¹H NMR (300 MHz, DMSO) δ 11.76 (s, 2H), 7.98 (t, J= 9.0 Hz, 2H).

### 1,4-dichloro-6,7-difluorophthalazine (xli)

This was prepared from (xxxii) according to the procedure given compound (xxiii) in example 18. Yield 52%. ¹H NMR (300 MHz, CDCl₃) δ 8.11 (t, J= 8.3 Hz, 2H). HRMS (ESI): cal. mass C₈H₂N₂F₂Cl₂ [M+1]⁺ 234.9636, [M+2]⁺ 236.9607 found [M+1]⁺ 234.9643, [M+2]⁺ 236.9615 cal. mass C₈H₂N₂F₂Cl₂Na [M+1]⁺ 256.9455, [M+2]⁺ 258.9426 found [M+Na]⁺ 256.9461, [M+2]⁺ 258.9430.

### 6-chloro-8,9-difluoro-3-methyl-[1,2,4]triazolo[3,4-a]phthalazine (xlii)

This was prepared from (xli) according to the procedure given in example 18. Yield (81%). ¹H NMR (300 MHz, CDCl₃) δ 8.45 (dd, J= 9.4, 7.3 Hz, 1H), 8.07 (dd, J= 9.4, 7.3 Hz, 1H), 2.81 (s, 3H). HRMS (ESI): cal. mass C₁₀H₅N₄F₂Cl [M+1]⁺ 255.0244, [M+2]⁺ 257.0214 found [M+1]⁺ 255.0246, [M+2]⁺ 257.0225 cal. mass C₁₀H₅N₄F₂Cl Na [M+1]⁺ 277.0063, [M+2]⁺ 279.0034 found [M+1]⁺ 277.0064, [M+2]⁺ 279.0034.

### 8,9-difluoro-3-methyl-[1,2,4]triazolo[3,4-a]phthalazine

This was prepared from compound (xlii) according to the procedure given in example 18. Yield (22%). ¹H NMR (300 MHz, CDCl₃) δ 8.56 (s, 1H), 8.44 (dd, J= 9.7, 7.3 Hz, 1H), 7.75 (dd, J= 9.2, 7.2 Hz, 1H). HRMS (ESI): cal. mass C₁₀H₆N₄F₂ [M+1]⁺ 221.0633, found [M+1]⁺ 221.0638, cal. mass C₁₀H₆N₄F₂ [M+Na]⁺ 243.0453, found [M+Na]⁺ 243.0463.

### Example 35

### 8,9-difluorotetrazolo[5,1-a]phthalazine 35

### 6-chloro-8,9-difluorotetrazolo[5,1-a]phthalazine (xliii)

This was prepared from compound (xli) according to the procedure given in example 18. Yield (49%). ¹H NMR (300 MHz, CDCl₃) δ 8.00 - 7.90 (m, 2H).

### 8,9-difluorotetrazolo[5,1-a]phthalazine 35

This was prepared from compound (xliii) according to the procedure given in example 18. Yield (15%). ¹H NMR (300 MHz, CDCl₃) δ 9.18 (s, 1H), 7.84 (d, J= 8.2 Hz, 1H), 7.13 (d, J= 8.2 Hz, 1H).

### Example 36

### 8,9-dichloro-3-methyl-[1,2,4]triazolo[3,4-a]phthalazine 36

### 5-(Trifluoromethyl)isobenzofuran-1(3H)-one (xliv)

4-(Trifluoromethyl)benzoic acid (5.0 g, 26.29 mmol, 1 equiv.), Pd(OAc)₂ (0.59 g, 2.62 mmol, 0.1 eq.), KH₂PO₄ (11.44 g, 65.74 mmol, 2.5 eq), and dibromomethane (30.0 mL) were charged in a 100 mL reaction tube with a magnetic stir bar. The reaction tube was sealed with a Teflon cap and the reaction mixture was stirred at 130 °C for 36 h. Then the mixture was filtered through a small pad of Celite, and the filtrate was concentrated under reduced pressure. The residue was purified by chromatography (silica gel, petroleum ether/ethyl acetate 20:1) to give the 5-(trifluoromethyl)-isobenzofuran-1(3H)-one (22%). ¹H NMR (300 MHz, CDCl₃) δ 8.06 (d, J= 7.9 Hz, 1H), 7.86 - 7.77 (m, 2H), 5.40 (s, 2H).

### 3-Bromo-5-(trifluoromethyl)isobenzofuran-1(3H)-one (xlv)

To a solution of 5-(trifluoromethyl)isobenzofuran-1(3H)-one (900 mg, 4.45 mmol, 1.0 equiv.) in dry benzene (20 mL) was added NBS (872 mg, 4.90 mmol, 1.1 eq) and AIBN (73 mg, 0.44 mmol, 0.1 eq.) at room temperature. Then the mixture was heated at reflux overnight. It was cooled to room temperature, concentrated under reduced pressure, and purified by chromatograph (silica gel, petroleum ether / ethyl acetate = 10/1) to give 3-bromo-5-(trifluoromethyl)isobenzofuran-1(3H)-one as a liquid (73%). ¹H NMR (300 MHz, CDCl₃) δ 8.07 (d, J= 8.1 Hz, 1H), 7.91 - 7.88 (m, 2H), 7.44 (s, 1H).

### 6-(Trifluoromethyl)phthalazin-1(2H)-one (xlvi)

3-Bromo-5-(trifluoromethyl)isobenzofuran-1(3H)-one (900 mg, 32.02 mmol, 1.0 equiv.) was dissolved in 10% HCl (20 mL), and hydrazine (55%) (240 mg, 4.80 mmol, 1.5 equiv.) was added and the mixture was refluxed with stirring for 16 h. The product was collected by suction filtration, washed with water and dried, to yield an amorphous solid (98%). ¹H NMR (300 MHz, DMSO) δ 12.92 (s, 1H), 8.50 (s, 1H), 8.45 - 8.37 (m, 2H), 8.14 (dd, *J* = 8.4, 1.8 Hz, 1H). HRMS (ESI): cal. mass C₉H₅F₃N₂O [M+1]⁺ 215.0427, found [M+1]⁺ 215.0434, cal. mass C₉H₅F₃N₂O [M+Na]⁺ 237.0246, found [M+Na]⁺ 237.0245.

### 1-Chloro-6-(trifluoromethyl)phthalazine (xlvii)

A round-bottomed flask was equipped with a stir bar and reflux condenser. To the flask was added the 6-(trifluoromethyl)phthalazin-1(2H)-one (600 mg, 2.80 mmol, 1.0 equiv.), phosphorus oxychloride (2.14 g, 14.00 mmol, 5.0 equiv.) and refluxed for 3 h. The reaction was cooled to r.t. and poured on ice. The mixture was separated and the aqueous layer was extracted with chloroform (4 × 60 mL). The organic layers were combined, dried (Na₂SO₄) and concentrated by rotary evaporation. The crude product was purified by column chromatography using dichloromethane give amorphous solid (49%). ¹H NMR (300 MHz, CDCl₃) δ 9.57 (s, 1H), 8.48 (d, J= 8.7 Hz, 1H), 8.33 (d, J= 1.7 Hz, 1H), 8.21 (dd, *J* = 8.7, 1.7 Hz, 1H). HRMS (ESI): cal. mass C₉H₄ClF₃N₂ [M+1]⁺ 233.0095, found [M+1]⁺ 233.0088, cal. mass C₉H₄ClF₃N₂ [M+Na]⁺ 254.9910, found [M+Na]⁺ 254.9907.

### 3-Methyl-8-(trifluoromethyl)-[1,2,4]triazolo[3,4-a]phthalazine (xlviii)

1-Chloro-6-(trifluoromethyl)phthalazine (60 mg, 0.26 mmol, 1.0 equiv.) and acetic acid hydrazide (28 mg, 0.38 mmol, 1.5 equiv.) were refluxed in dioxane (3 mL) for 16 h then concentrated under reduced pressure. The reaction mixture was extracted with dichloromethane (4 × 40 mL). The organic phase was dried with Na₂SO₄ and concentrated by rotary evaporation. The crude product was purified by column chromatography using dichloromethane give amorphous solid **36** (70%). ¹H NMR (300 MHz, CDCl₃) δ 8.80 (d, J= 8.4 Hz, 1H), 8.70 (s, 1H), 8.22 (d, *J=* 1.7 Hz, 1H), 8.17 (dd, *J =* 8.4, 1.7 Hz, 1H), 2.86 (s, 3H). ¹⁹F NMR (282 MHz, CDCl₃) δ -62.80. HRMS (ESI): cal. mass C₁₁H₇F₃N₄ [M+1]⁺ 253.0695, found [M+1]⁺ 253.0696, cal. mass C₁₁H₇F₃N₄ [M+Na]⁺ 275.0516, found [M+Na]⁺ 275.0515.

### Example 37

### 8-(Trifluoromethyl)tetrazolo[5,1-a]phthalazine 37

1-Chloro-6-(trifluoromethyl)phthalazine (60 mg, 0.26 mmol, 1.0 equiv.) and sodium azide (25 mg, 0.38 mmol, 1.5 equiv.) were refluxed in acetonitrile (3 mL) for 16 h then concentrated under reduced pressure. The reaction mixture was extracted with dichloromethane (4 × 40 mL). organic phase was dried with Na₂SO₄ and concentrated by rotary evaporation. The crude product was purified by column chromatography using dichloromethane give amorphous solid (69%). ¹H NMR (300 MHz, CDCl₃) δ 9.06 (s, 1H), 8.92 (d, J= 8.4 Hz, 1H), 8.46 (d, *J=* 1.7 Hz, 1H), 8.35 (dd, *J=* 8.4, 1.7 Hz, 1H). ¹⁹F NMR (282 MHz, CDCl₃) δ -62.93. HRMS (ESI): cal. mass C₉H₄F₃N₅ [M+1]⁺ 240.0492, found [M+1]⁺ 240.0492, cal. mass C₉H₄F₃N₅ [M+Na]⁺ 262.0312, found [M+Na]⁺ 262.0311.

### Example 38

### 8-(Trifluoromethyl)-[1,2,4]triazolo[3,4-a]phthalazine 38

1-Chloro-6-(trifluoromethyl)phthalazine (60 mg, 0.26 mmol, 1.0 equiv.) and formic acid hydrazide (23 mg, 0.38 mmol, 1.5 equiv.) were refluxed in acetonitrile (3 mL) for 16 h then concentrated under reduced pressure. The reaction mixture was extracted with dichloromethane (4 × 40 mL). The organic phase was dried with Na₂SO₄ and concentrated by rotary evaporation. The crude product was purified by column chromatography using dichloromethane give amorphous solid **38** (69%). ¹H NMR (300 MHz, CDCl₃) δ 9.12 (s, 1H), 8.85 (d, *J=* 8.3 Hz, 1H), 8.74 (s, 1H), 8.24 (s, 1H), 8.20 (d, J= 8.3 Hz, 1H). ¹⁹F NMR (282 MHz, CDCl₃) δ -62.84. HRMS (ESI): cal. mass C₁₀H₅F₃N₄ [M+1]⁺ 239.0545, found [M+1]⁺ 239.0539, cal. mass C₁₀H₅F₃N₄ [M+Na]⁺ 261.0357, found [M+Na]⁺ 261.0359.

### Example 39

### 9-Methoxy-3-methyl-[1,2,4]triazolo[3,4-a]phthalazine 39

### 6-Methoxy-2,3-dihydrophthalazine-1,4-dione (xlix)

To a solution of 4,5-dichlorophthalic acid (5 g, 25.48 mmol, 1.0 equiv.) in Ac₂O (40 mL) was refluxed for 2 h and then concentrated to afford a light brown solid 5-methoxyisobenzofuran-1,3-dione and the crude product was directly subjected to the next reaction without further purification. To a solution of 5-methoxyisobenzofuran-1,3-dione in ethanol (40 mL) hydrazine (55%) (1.63 g, 50.97 mmol, 2.0 equiv.) was added and the mixture was refluxed and stirred for 24 h. The product was collected by suction filtration, washed with water and dried, to yield as an amorphous solid (xlix) (4.1 g, 85%).

### 1,4-Dichloro-6-methoxyphthalazine (I)

A round-bottomed flask was equipped with a stir bar and reflux condenser. To the flask was added the 6-methoxy-2,3-dihydrophthalazine-1,4-dione (xlix) (4.0 g, 20.82 mmol, 1.0 equiv.), phosphorus oxychloride (15.92 g, 104.11 mmol, 5.0 equiv.) and refluxed for 4 h. The reaction was cooled to rt and poured over ice. The mixture was separated and the aqueous layer was extracted with chloroform (4 × 70 mL). The organic layers were combined, dried (Na₂SO₄) and concentrated by rotary evaporation. The crude product was purified by column chromatography using dichloromethane give amorphous solid (I) (1.35 g, 28%). ¹H NMR (300 MHz, CDCl₃) δ 8.20 (d, J= 9.1 Hz, 1H), 7.60 (dd, *J =* 9.1, 2.5 Hz, 1H), 7.49 (d, J= 2.5 Hz, 1H), 4.06 (s, 3H). ¹³C NMR (75 MHz, CDCl₃) δ 164.03, 154.21, 129.49, 127.99, 126.18, 122.17, 121.10, 104.18, 56.29. HRMS (ESI): cal. mass C₉H₆N₂OCl₂ [M+1]⁺ 228.9930, [M+2]⁺ 230.9901 found [M+1]⁺ 228.9933, [M+2]⁺ 230.9908 cal. mass C₉H₆N₂OCl₂Na [M+1]⁺ 250.9749, [M+2]⁺ 252.9720 found [M+1]⁺ 250.9757, [M+2]⁺ 252.9730.

### 6-Chloro-9-methoxy-3-methyl-[1,2,4]triazolo[3,4-a]phthalazine (ii) and 6-chloro-8-methoxy-3-methyl-[1,2,4]triazolo[3,4-a]phthalazine (iii)

This was prepared from 1,4-dichloro-6-methoxyphthalazine (I) according to the procedure given in example 14. Yield (86%). ¹H NMR (300 MHz, CDCl₃) δ 8.58 (d, *J* = 8.7 Hz, 0.7H), 8.14 (d, *J=* 9.1 Hz, 1H), 8.02 (d, *J=* 2.6 Hz, 1H), 7.61 - 7.52 (m, 1.5 H), 7.35 (dd, *J =* 9.1, 2.6 Hz, 1H), 4.05 (s, 3H), 4.01 (s, 2H), 2.81 (s, 3H), 2.79 (s, 2H).

### 9-Methoxy-3-methyl-[1,2,4]triazolo[3,4-a]phthalazine 39

This was prepared from (li/lii) according to the procedure given in example 14. Yield (21%). Mixture of isomers were separated by column chromatography using DCM and MeOH (98:2). First isomer 9-methoxy-3-methyl-[1,2,4]triazolo[3,4-a]phthalazine **39.** ¹H NMR (600 MHz, CDCl₃) δ 8.53 (s, 1H), 8.06 (d, J= 2.5 Hz, 1H), 7.83 (d, J= 8.7 Hz, 1H), 7.33 (dd, J= 8.7, 2.4 Hz, 1H), 4.04 (s, 3H), 2.83 (s, 3H). ¹³C NMR (126 MHz, CDCl₃) δ 164.11, 148.24, 146.92, 143.02, 130.03, 125.75, 121.21, 117.26, 104.03, 56.43, 10.09.

### Example 40

### 8-Methoxy-3-methyl-[1,2,4]triazolo[3,4-a]phthalazine 40

This was prepared from (li/lii) according to the procedure given in example 14. Yield (21%). Mixture of isomers were separated by column chromatography using DCM and MeOH (98:2). Second isomer 8-methoxy-3-methyl-[1,2,4]triazolo[3,4-a]phthalazine **40.** ¹H NMR (600 MHz, CDCl₃) δ 8.57 (d, J= 8.8 Hz, 1H), 8.55 (s, 1H), 7.52 (dd, J= 8.8, 2.5 Hz, 1H), 7.28 (d, J= 2.5 Hz, 1H), 3.98 (s, 3H), 2.81 (s, 3H). ¹³C NMR (126 MHz, CDCl₃) δ 161.31, 147.65, 146.92, 142.74, 125.01, 124.63, 122.98, 117.27, 109.38, 55.80, 9.90.

### Example 41

### 9-Chloro-6-isopropoxy-3-methyl[1,2,4]triazolo[3,4-a]phthalazine 41a and 8-chloro-6-isopropoxy-3-methyl-[1,2,4]triazolo[3,4-a]phthalazine 41b

Compound (xx/xxi) from example 21 (100 mg, 0.39 mmol, 1.0 equiv.) and K₂CO₃ (109 mg, 0.79 mmol, 2.0 equiv.) were refluxed in isopropanol (5 mL) for 36 h then concentrated under reduced pressure. The reaction mixture was extracted with dichloromethane (4 × 40 mL). The organic phase was dried with Na₂SO₄ and concentrated by rotary evaporation. The crude product was purified by column chromatography using dichloromethane give amorphous solid as mixture **41a/41b** (89%). ¹H NMR (300 MHz, CDCl₃) δ 8.53 (d, *J* = 2.1 Hz, 1H), 8.50 (d, J= 8.5 Hz, 1H), 8.12 (d, J= 2.1 Hz, 1H), 8.08 (d, J= 8.7 Hz, 1H), 7.82 (dd, J= 8.5, 2.1 Hz, 1H), 7.65 (dd, J= 8.6, 2.1 Hz, 1H), 5.45 (hept, J= 6.2 Hz, 2H), 2.72 (s, 6H), 1.52 (d, *J* = 6.2 Hz, 12H). HRMS (ESI): cal. mass C₁₃H₁₃N₄OCl [M+1]⁺ 277.0851, [M+2]⁺ 279.0822 found [M+1]⁺ 277.0850, [M+2]⁺ 279.0824 cal. mass C₁₃H₁₃N₄OCl Na [M+1]⁺ 299.0670, [M+2]⁺ 301.0641 found [M+1]⁺ 299.0670, [M+2]⁺ 301.0639.

### Examples 42 and 43

### 9-chloro-6-isopropoxytetrazolo[5,1-a]phthalazine 42 and 8-chloro-6-isopropoxytetrazolo[5,1-a]phthalazine 43

This was prepared from compounds 6,9-dichlorotetrazolo[5,1-a]phthalazine (xxii) and 6,8-dichlorotetrazolo[5,1-a]phthalazine (xxiii) from example 23 according to the procedure given in example 41 as mixture of isomers. Yield (81%). ¹H NMR (300 MHz, CDCl₃) δ 8.62 (d, J= 2.1 Hz, 0.5H), 8.58 (d, J= 8.5 Hz, 1H), 8.28 (d, J= 2.1 Hz, 1H), 8.25 (d, J= 8.8 Hz, 0.5H), 7.98 (dd, J= 8.5, 2.1 Hz, 1H), 7.86 (dd, J= 8.7, 2.1 Hz, 0.5H), 5.63 (hept, J= 6.2, 1.5H), 1.57 (d, *J* = 6.2 Hz, 9H). HRMS (ESI): cal. mass C₁₁H₁₀N₅OCl [M+1]⁺ 264.0647, [M+2]⁺ 266.0618 found [M+1]⁺ 264.0647, [M+2]⁺ 266.0613 cal. mass C₁₁H₁₀N₅OCl Na [M+1]⁺ 286.0466, [M+2]⁺ 288.0437 found [M+1]⁺ 286.0465, [M+2]⁺ 288.0438.

Isomers were separated by column chromatography using DCM.

9-chloro-6-isopropoxytetrazolo[5,1-a]phthalazine **42** (first isomer) ¹H NMR (300 MHz, CDCl₃) δ 8.62 (d, J= 2.1 Hz, 1H), 8.25 (d, J= 8.7 Hz, 1H), 7.86 (dd, J= 8.7, 2.1 Hz, 1H), 5.62 (hept, J= 6.2 Hz, 1H), 1.57 (d, J= 6.2 Hz, 6H). ¹³C NMR (75 MHz, CDCl₃) δ 157.59, 141.07, 140.36, 132.74, 127.41, 124.13, 123.96, 118.90, 73.59, 21.70.

8-chloro-6-isopropoxytetrazolo[5,1-a]phthalazine **43** (second isomer) ¹H NMR (300 MHz, CDCl₃) δ 8.58 (d, J= 8.5 Hz, 1H), 8.27 (d, J= 2.1 Hz, 1H), 7.98 (dd, J= 8.5, 2.1 Hz, 1H), 5.62 (hept, J= 6.2 Hz, 1H), 1.57 (d, J= 6.1 Hz, 6H). ¹³C NMR (75 MHz, CDCl₃) δ 157.73, 140.46, 138.85, 134.79, 126.57, 125.53, 121.87, 121.10, 72.67, 22.59.

### Example 44

### 10-chloro-6-isopropoxytetrazolo[5,1-a]phthalazine 44

This was prepared from compound 6,10-dichlorotetrazolo[5,1-a]phthalazine (xvi) in example 19 according to the procedure given in example 41. Yield (86%). ¹H NMR (300 MHz, CDCl₃) δ 8.27 (d, J= 8.1Hz, 1H), 8.05 (d, J= 8.1 Hz, 1H), 7.84 (t, J= 8.1 Hz, 1H), 5.63 (hept, J= 6.1 Hz, 1H), 1.57 (d, J= 6.1 Hz, 6H). ¹³C NMR (126 MHz, CDCl₃) δ 158.36, 139.91, 135.85, 132.66, 132.37, 124.38, 122.56, 121.70, 73.84, 21.83. HRMS (ESI): cal. mass C₁₁H₁₀N₅OCl [M+1]⁺ 264.0647, [M+2]⁺ 266.0618 found [M+1]⁺ 264.0645, [M+2]⁺ 266.0616 cal. mass C₁₁H₁₀N₅OCl Na [M+1]⁺ 286.0466, [M+2]⁺ 288.0437 found [M+1]⁺ 286.0464, [M+2]⁺ 288.0438.

### Example 45

### 6-Isopropoxy-3-methyl-[1,2,4]triazolo[3,4-a]phthalazine 45

This was prepared from 6-chloro-3-methyl-1,2,4-triazolo[3,4-a]phthalazine (i) in example 3 according to the procedure given in example 46 for 6-chlorotetrazolo[5,1-a]phthalazine. Yield (91%). ¹H NMR (300 MHz, CDCl₃) δ 8.56 (dd, *J* = 8.0, 1.2 Hz, 1H), 8.15 (dd, *J* = 8.0, 1.2 Hz, 1H), 7.86 (td, *J* = 7.7, 1.3 Hz, 1H), 7.72 (td, *J* = 7.7, 1.3 Hz, 1H), 5.45 (hept, *J* = 6.2 Hz, 1H), 2.72 (s, 3H), 1.52 (d, *J* = 6.2 Hz, 6H). ¹³C NMR (126 MHz, CDCl₃) δ 157.16, 147.60, 142.46, 133.44, 130.23, 125.28, 124.74, 123.10, 119.17, 71.53, 21.84, 9.87. HRMS (ESI): cal. mass C₁₃H₁₄N₄O [M+1]⁺ 243.1240, found [M+1]⁺ 243.1241, cal. mass C₁₃H₁₄N₄O [M+Na]⁺ 265.1067, found [M+Na]⁺ 265.1060.

### Example 46

### 6-Isopropoxytetrazolo[5,1-a]phthalazine 46

### 6-Chlorotetrazolo[5,1-a]phthalazine (liii)

1-Chloro-4-hydrazinylphthalazine (400 mg, 2.405 mmol, 1.0 equiv.) was dissolved in acetic acid (20 mL, 2N) and under stirring the ice-cold solution was treated with a cold aqueous solution of NaNO₂ (212 mg, 3.08 mmol, 1.5 equiv. in 2 mL water) dropwise. The reaction mixture was stirred at room temperature for 1 h. The compound was extracted with chloroform (3 X 40 mL). Organic phase was dried with Na₂SO₄ and evaporated to give an amorphous solid 6-chlorotetrazolo[5,1-a]phthalazine (335 mg, 80%). ¹H NMR (300 MHz, CDCl₃) δ 8.78 (dd, *J* = 7.8, 1.4 Hz, 1H), 8.45 (dd, *J* = 7.8, 1.4 Hz, 1H), 8.18 (td, *J* = 7.8, 1.4 Hz, 1H), 8.09 (td, *J* = 7.8, 1.4 Hz, 1H). ¹³C NMR (75 MHz, CDCl₃) δ 152.25, 141.89, 135.67, 133.37, 128.04, 125.11, 124.17, 122.37.

6-Chlorotetrazolo[5,1-a]phthalazine (liii) (200 mg, 0.97 mmol, 1.0 equiv.) and K₂CO₃ (268 mg, 1.94 mmol, 2.0 equiv.) were refluxed in isopropanol (8 mL) for 36 h then concentrated under reduced pressure. The reaction mixture was extracted with dichloromethane (4 x 40 mL). Organic phase was dried with Na₂SO₄ and concentrated by rotary evaporation. The crude product was purified by column chromatography using dichloromethane give amorphous solid **46** (86%). ¹H NMR (300 MHz, CDCl₃) δ 8.62 (dd, *J* = 8.1, 1.3 Hz, 1H), 8.31 (dd, *J* = 8.1, 1.3 Hz, 1H), 8.01 (td, *J* = 7.7, 1.3 Hz, 1H), 7.92 (td, *J* = 7.7, 1.3 Hz, 1H), 5.62 (hept, *J* = 6.1 Hz, 1H), 1.56 (d, *J* = 6.1 Hz, 6H). ¹³C NMR (126 MHz, CDCl3) δ 158.87, 141.07, 134.31, 132.26, 125.86, 124.56, 122.86, 120.82, 73.30, 21.86. HRMS (ESI): cal. mass C₁₁H₁₁N₅O [M+1]⁺ 230.1036, found [M+1]⁺ 230.1038, cal. mass C₁₁H₁₁N₅O [M+Na]⁺ 252.0856, found [M+Na]⁺ 252.0857.

### Biological results

### Biological results

### Example 47

### Cell culture Human Dermal Fibroblasts

**Compounds reconstitution and stock solution:** Prior to treatment, DMSO was diluted in PMS until 0,1% DMSO was made. Each compound was solved in 0,1% DMSO **(3:** 2,5 mg/mL, **1:** 2.5 mg/mL, **10:** 1 mg/mL, **5:** 1mg/mL, **7:** 0,6 mg/mL, **16:** 1 mg/mL **17:** 1 mg/mL, **44:** 0,6 mg/mL, **36:** 1mg/mL.

Next the different stock solution with 150 µg and 250 µg of each compound per 1 ml culture solution was made by adding the 0,1% DMSO compound solution in the right amount to culture solution (DMEM with 10% FCS and 1% penicillin-streptomycin).

### Cell lines

Three different cell lines were used for the experiments. GM637 is an immortalized cell line originated from human skin fibroblasts, bought in 2001, UKE1 and HRO59N are cell lines originated from human dermal fibroblasts (dog ear) nurtured from patients, between 60 and 80 years old being completely anonymized at the clinic for dermatology, Rostock.

### Cell culture

The cells lines were thawed and were cultured in Dulbeco's modified Eagel's medium (DMEM, Gibcon, USA) supplemented with 10% fetal bovine serum (FBS, Gibco, Germany) and 1% penicillin-streptomycin (PS, Gibco, USA) at 37 °C in a humidified atmosphere with 5% CO₂ until a sufficient number of cells were culture in T75 culture bottles. After reaching a confluence of 80% to 90%, the cells were separated using trypsin and counted. Next 100.000 cells were planted in each well of a 6 well plate in replicates, washed and the DMEM solution with 150 µg and 250 µg/ml substance/DMEM and DMEM with no substance as control was added.

After 48h and after 72h 100 µl supernatant of each well was taken, labelled and frozen at -20° degree Celsius. Prior to perform ELISA the medium was depending on the cell line diluted 1:50 or 1:100 with PBS, otherwise the concentration of collagen fibers was too high to be measured.

### Biological results

### Example 48

### Cell culture Human Lung Fibroblasts

**Compounds reconstitution and stock solution:** Prior to treatment, DMSO was diluted in PMS until 0,1% DMSO was made. Each compound was solved in 0,1% DMSO (**3:** 2,5 mg/mL, **1:** 2.5 mg/mL, **10:** 1 mg/mL, **5:** 1mg/mL, **7:** 0,6 mg/mL, **16:** 1 mg/mL **17:** 1 mg/mL, **44:** 0,6 mg/mL, **36:** 1mg/mL.

Next the different stock solution with 150 µg and 250 µg of each compound per 1 ml culture solution was made by adding the 0,1% DMSO compound solution in the right amount to culture solution (DMEM with 10% FCS and 1% penicillin-streptomycin).

### Cell line

As cell line MRCV1 was used, which is an immortalized cell line originated from human lung fibroblast, bought in 2001.

### Cell culture

The cell line was thawed and cultured in Dulbeco's modified Eagel's medium (DMEM, Gibcon, USA) supplemented with 10% fetal bovine serum (FBS, Gibco, Germany) and 1% penicillin-streptomycin (PS, Gibco, USA) at 37 °C in a humidified atmosphere with 5% CO₂ until a sufficient number of cells were culture in T75 culture bottles. After reaching a confluence of 80% to 90%, the cells were separated using trypsin and counted. Next 100.000 cells were planted in each well of a 6 well plate in replicates, washed and the DMEM solution with 150 µg and 250 µg/ml substance/DMEM and DMEM with no substance as control was added.

After 48h and after 72h 100 µl supernatant of each well was taken, labelled and frozen at -20° degree Celsius. Prior to perform ELISA the medium was depending on the cell line diluted 1:50 or 1:100 with PBS, otherwise the concentration of collagen fibers was too high to be measured.

### Example 49

### Enzyme-linked immunosorbent assay (ELISA) to detect secreted Type I Collagen from human dermal fibroblasts

The media containing the secretion of the different human dermal fibroblasts GM637, UKE1, and HRO159N were collected after 48h and 72h treatment. Secretion of pro-collagen 1 was assessed with human pro-collagen I alpha 1 DuoSet ELISA (R&D systems, USA) To prepare the Elisa plate, Human pro-Collagen I a capture antibody was diluted in ELISA plate-coating buffer at 4µg/mL working concentration. 100 µl of diluted Capture antibody was added to 96-well microplates and incubated over night at room temperature. After three times washing with Wash Buffer, microplates were blocked by adding 300 µl of Reagent Diluent for 1 hour at room temperature. To proceed the ELISA Human pro-collagen I α1 Standards were diluted in Reagent Diluent (range between 31,3 pg/mL - 2000 pg/mL) and 100 µl of standards or conditioned mediums were added to designated wells. Microplates were incubated at room temperature for 2 hours. Human Pro-collagen I α1 Detection Antibody was prepared in Reagent Diluent at ng/mL working concentration and 100 µl of diluted Detection Antibody was applied for 2 hours at room temperature after extensive washing. Then, working solution of Streptavidin-HRP with Reagent Diluent was prepared by diluting 1:40 and added into each well. After 20 minutes at room temperature incubation, microplates were washed and applied with 100 µl of Substrate Solution for 20 minutes followed by Stop Solution after washing. Finally, signals were measured at 450 nm absorbance by PHomo Microplate Reader (Autobio, China) The amount of pro-collagen 1 was quantified by extrapolating the signal into a linear range of a pro-collagen 1 standard curve. Screening compounds were typically tested at concentration of 150 and 250 µg/mL. The dose response data for each compound and the different cell lines were obtained by nonlinear regression analysis using Prism 9 software (GraphPad, La Jolla, USA). The collagen reduction percentage in comparison to the standard (100 %) regarding the human fibroblasts cell lines and the compounds **1, 3, 5, 7, 16, 17** and **44** for the application time of 48h and 72 h and for the concentrations 150 µg/250 µg is given below:
49/1: GM637: **3**/ 48h/ 150 µg: 89% /250 µg: 50% // 72 h/150 µg: 37% /250 µg: 32%
49/2: GM637: **1**/ 48h/ 150 µg: 127% /250 µg: 77% // 72 h/150 µg: 95% /250 µg: 92%
49/3: GM637: **5**/ 48h/ 150 µg: 114% /250 µg: 89% // 72 h/150 µg: 70% /250 µg: 105%
49/4: GM637: **7**/ 48h/ 150 µg: 101% /250 µg: 75% // 72 h/150 µg: 43% /250 µg: 30%
49/5: GM637: **16**/ 48h/ 150 µg: 79% /250 µg: 62% // 72 h/150 µg: 86% /250 µg: 55%
49/6: GM637: **17**/ 48h/ 150 µg: 90% /250 µg: 62% // 72 h/150 µg: 103% /250 µg: 55%
49/7: GM637: **44**/ 48h/ 150 µg: 122% /250 µg: 89% // 72 h/150 µg: 61% /250 µg: 65%
49/8: UKE1: **3**/ 48h/ 150 µg: 96% /250 µg: 77% // 72 h/150 µg: 71% /250 µg: 52%
92/9: UKE1: **1**/ 48h/ 150 µg: 70% /250 µg: 85% // 72 h/150 µg: 74% /250 µg: 68%
49/10: UKE1: **5**/ 48h/ 150 µg: 107% /250 µg: 99% // 72 h/150 µg: 94% /250 µg: 62%
49/11: UKE1: **7**/ 48h/ 150 µg: 79% /250 µg: 54% // 72 h/150 µg: 44% /250 µg: 47%
49/12: UKE1: **16**/ 48h/ 150 µg: 117% /250 µg: 99% // 72 h/150 µg: 82% /250 µg: 35%
49/13: UKE1: **17**/ 48h/ 150 µg: 102% /250 µg: 86% // 72 h/150 µg: 76% /250 µg: 59%
49/14: UKE1: **44**/ 48h/ 150 µg: 122% /250 µg: 89% // 72 h/150 µg: 61% /250 µg: 65%
49/15: HRO159N: **3**/ 48h/ 150 µg: - /250 µg: 82% // 72 h/150 µg: - /250 µg: 77%
49/16: HRO159N: **1**/ 48h/ 150 µg: -/250 µg: 57% // 72 h/150 µg: - /250 µg: 72%
49/17: HRO159N: **7**/ 48h/ 150 µg: - /250 µg: 43% // 72 h/150 µg: -% /250 µg: 33%
49/18: HRO159N: **16**/ 48h/ 150 µg: - /250 µg: 79% // 72 h/150 µg: - /250 µg: 48%
49/19: HRO159N: **17**/ 48h/ 150 µg: - /250 µg: 34% // 72 h/150 µg: - /250 µg: 4%
49/20: HRO159N: **44**/ 48h/ 150 µg: - /250 µg: 72% // 72 h/150 µg: - /250 µg: 79%

### Example 50

### Enzyme-linked immunosorbent assay (ELISA) to detect secreted Type I Collagen from human lung fibroblasts

The medium containing the secretion of the human lung fibroblast MRCVI was collected after 48h and 72h treatment. Secretion of pro-collagen 1 was assessed with human pro-collagen I alpha 1 DuoSet ELISA (R&D systems, USA) To prepare the Elisa plate, Human pro-Collagen I a capture antibody was diluted in ELISA plate-coating buffer at 4µg/mL working concentration. 100 µl of diluted Capture antibody was added to 96-well microplates and incubated over night at room temperature. After three times washing with Wash Buffer, microplates were blocked by adding 300 µl of Reagent Diluent for 1 hour at room temperature. To proceed the ELISA Human pro-collagen I α1 Standards were diluted in Reagent Diluent (range between 31,3 pg/mL - 2000 pg/mL) and 100 µl of standards or conditioned mediums were added to designated wells. Microplates were incubated at room temperature for 2 hours. Human Pro-collagen I α1 Detection Antibody was prepared in Reagent Diluent at ng/mL working concentration and 100 µl of diluted Detection Antibody was applied for 2 hours at room temperature after extensive washing. Then, working solution of Streptavidin-HRP with Reagent Diluent was prepared by diluting 1:40 and added into each well. After 20 minutes at room temperature incubation, microplates were washed and applied with 100 µl of Substrate Solution for 20 minutes followed by Stop Solution after washing. Finally, signals were measured at 450 nm absorbance by PHomo Microplate Reader (Autobio, China) The amount of pro-collagen 1 was quantified by extrapolating the signal into a linear range of a pro-collagen 1 standard curve. Screening compounds were typically tested at concentration of 150 and 250 µg/mL. The dose response data for each compound and the different cell lines were obtained by nonlinear regression analysis using Prism 9 software (GraphPad, La Jolla, USA). The collagen reduction percentage in comparison to the standard (100 %) regarding the human lung fibroblast cell line and the compounds **1, 3, 5, 7, 10, 16, 17, 36** and **44** for the application time of 48h and 72 h and for the concentrations 150 µg/250 µg is given below:
50/1: MRCVI: **3**/ 48h/ 150 µg: 35% /250 µg: 11% // 72 h/150 µg: 17% /250 µg: 5%
50/2: MRCVI: **1**/ 48h/ 150 µg: 87% /250 µg: 78% // 72 h/150 µg: 74% /250 µg: 61%
50/3: MRCVI: **5**/ 48h/ 150 µg: 97% /250 µg: 107% // 72 h/150 µg: 56% /250 µg: 108%
50/4: MRCVI: **7**/ 48h/ 150 µg: 19% /250 µg: 9% // 72 h/150 µg: 14% /250 µg: 3%
50/5: MRCVI: **16**/ 48h/ 150 µg: 72% /250 µg: 43% // 72 h/150 µg: 82% /250 µg: 35%
50/6: MRCVI: **17**/ 48h/ 150 µg: 33% /250 µg: 16% // 72 h/150 µg: 35% /250 µg: 11%
50/7: MRCVI: **36**/48h/150 µg: 64% /250 µg: 68% // 72 h/150 µg: 36% /250 µg: 22%
50/8: MRCVI: **10**/48h/150 µg: 67% /250 µg: 52% // 72 h/150 µg: 48% /250 µg: 25%

### Cited literature:

### References

1. Papara C, De Luca DA, Bieber K et al. Morphea: The 2023 update. Front Med (Lausanne) 2023; 10: 1108623. DOI: 10.3389/fmed.2023.1108623.
2. Peterson LS, Nelson AM, Su WP et al. The epidemiology of morphea (localized scleroderma) in Olmsted County 1960-1993. J Rheumatol 1997; 24: 73-80.
3. Saracino AM, Kelberman D, Otto GW et al. Unravelling morphoea aetiopathogenesis by next-generation sequencing of paired skin biopsies. Arch Dermatol Res 2023. DOI: 10.1007/s00403-023-02541-5.
4. Fett N, Werth VP. Update on morphea: part I. Epidemiology, clinical presentation, and pathogenesis. J Am Acad Dermatol 2011; 64: 217-228; quiz 229-230. DOI: 10.1016/j.jaad.2010.05.045.
5. Fett N, Werth VP. Update on morphea: part II. Outcome measures and treatment. J Am Acad Dermatol 2011; 64: 231-242; quiz 243-234. DOI: 10.1016/j.jaad.2010.05.046.
6. Bhattacharyya S, Wei J, Tourtellotte WG et al. Fibrosis in systemic sclerosis: common and unique pathobiology. Fibrogenesis Tissue Repair 2012; 5: S18. DOI: 10.1186/1755-1536-5-S1-S18
7. Brinckmann J, Neess CM, Gaber Y et al. Different pattern of collagen cross-links in two sclerotic skin diseases: lipodermatosclerosis and circumscribed scleroderma. J Invest Dermatol 2001; 117: 269-273. doi: 10.1046/j.0022-202x.2001.01414.x.
8. Kreuter A, Krieg T, Worm M et al [AWMF Guideline no. 013/066. Diagnosis and therapy of circumscribed scleroderma]. J Dtsch Dermatol Ges 2009; 7 Suppl 6: S1-14. DOI: 10.1111/j.1610-0387.2009.07178.x. 7
9. Hernandez-Rodriguez JC, Sendin-Martin M, Duran-Romero AJ et al. Systemic sclerosis mortality trends in Spain from 1980 to 2019: age-period-cohort and joinpoint analysis. Clin Exp Dermatol 2022; 47: 1943-1950. doi: 10.1111/ced.15342
10. Westerlind H, Bairkdar M, Gunnarsson K et al. Incidence and prevalence of systemic sclerosis in Sweden, 2004-2015, a register-based study. Semin Arthritis Rheum 2022; 53: 151978. DOI: 10.1016/j.semarthrit.2022.151978.
11. Jerjen R, Nikpour M, Krieg T et al. Systemic sclerosis in adults. Part I: Clinical features and pathogenesis. J Am Acad Dermatol 2022; 87: 937-954. DOI: 10.1016/j.jaad.2021.10.065.
12. Zanin-Silva DC, Santana-Goncalves M, Kawashima-Vasconcelos MY et al. Management of Endothelial Dysfunction in Systemic Sclerosis: Current and Developing Strategies. Front Med (Lausanne) 2021; 8: 788250. DOI: 10.3389/fmed.2021.788250.
13. Mouawad JE, Feghali-Bostwick C. The Molecular Mechanisms of Systemic Sclerosis-Associated Lung Fibrosis. Int J Mol Sci 2023; 24. DOI: 10.3390/ijms24032963.
14. Distler O, Highland KB, Gahlemann M et al. Nintedanib for Systemic Sclerosis-Associated Interstitial Lung Disease. N Engl J Med 2019; 380: 2518-2528. DOI: 10.1056/NEJMoa1903076.
15. Khanna D, Lin CJF, Furst DE et al. Tocilizumab in systemic sclerosis: a randomised, double-blind, placebo-controlled, phase 3 trial. Lancet Respir Med 2020; 8: 963-974. doi: 10.1016/52213-2600(20)30318-0.
16. Druey, J., and B. H. Ringier. "Hydrazinderivate der Phtalazin-und Pyridazinreihe." Helvetica Chimica Acta 34.1 (1951): 195-210.
17. Zimmer, Hans, John M. Kokosa, and K. J. Shah. "Synthesis of condensed heterocyclic systems. VI. Ring closure reactions involving 1-hydrazinophthalazine." The Journal of Organic Chemistry 40.20 (1975): 2901-2906.
18. Badr, M. Z. A., et al. "Substitution and ring closure reactions of phthalazine derivatives. "Journal of heterocyclic chemistry 21.2 (1984): 471-475*.*

## Claims

1. Pyridazine compound (I) 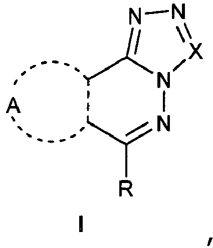 wherein
R is selected from the group consisting of H, D, OR³, and C₁₋₄-alkyl, in particular H, D, methyl;
X is N or CR¹, wherein the nitrogen or carbon atom is part of an aromatic ring system;
A is selected from the group consisting of CR²=CR²-CR²=CR², S-CR²=CR² or CR²-S-CR² , thus forming either a benzopyridazine (phthalazine) or a thienopyridazine compound, wherein the carbon atom is part of an aromatic ring system and wherein 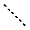 as part of the pyridazine ring denotes a single bond in case of CR²-S-CR² or a double bond in case of CR²=CR²-CR²=CR² or S-CR²=CR²; and wherein 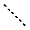 as part of ring A denotes a single bond in case of CR²=CR²-CR²=CR² or S-CR²=CR² or a double bond in case of CR²-S-CR², and wherein
R¹ is selected from the group consisting of H, D, CH₃, CD₃, CHD₂, CH₂D, CF₃, CHF₂, CH₂F, CDF₂, CD₂F, C₁₋₄-alkyl, optionally substituted with OH, halogen, OR³;
R² is selected from the group consisting of H, D, F, Cl, methyl, CH₂F, CHF₂, CF₃, OCH₃, OCF₃, OCD₃ when X denotes CR¹ and R² is selected from the group consisting of H, D, F, Cl, CH₂F, CHF₂, CF₃ when X denotes N.
R³ is selected from the group consisting of C₁-₄-alkyl, for use in a method of treatment or prevention of skin or lung fibrosis.

2. The pyridazine compound (I) for use according to claim 1, wherein R is selected from the group consisting of H, D, OR³, and wherein R¹ is selected from the group H, D, CH₃, CD₃, CF₃.

3. The pyridazine compound (I) for use according to any of the claims 1 and 2, wherein A is selected from the group consisting of S-CH=CH or CH-S-CH, thus forming a thienopyridazine compound, wherein 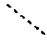 as part of the pyridazine ring denotes a single bond in case of CH-S-CH or a double bond in case of S-CH=CH, and wherein R is H, and wherein R¹ is selected from the group consisting of H, D, CH₃, CD₃, when X denotes CR¹ or X denotes N.

4. The pyridazine compound (I) for use to any of the claims 1-3, wherein A is selected from a group CR²=CR²-CR²=CR², thus forming either a benzopyridazine (phthalazine) wherein 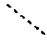 as part of the pyridazine ring denotes a double bond and wherein 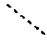 as part of ring A denotes a single bond, and wherein X denotes a CCH₃ group, and wherein one R² is selected from the group consisting of H, D, F, Cl, methyl, CF₃, OCF₃, OCH₃, and the remaining R² are hydrogens; and wherein R is hydrogen.

5. The pyridazine compound (I) for use to any of the claims 1-4, wherein A is selected from a group CR²=CR²-CR²=CR²,
thus forming either a benzopyridazine (phthalazine) wherein 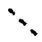 as part of the pyridazine ring denotes a double bond and wherein 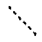 as part of ring A denotes a single bond, and wherein X denotes a CCH₃ group, and wherein two R² are independently from each other selected from the group consisting of H, D, F, Cl, methyl, CF₃, OCF₃, OCH₃, and the remaining R² are hydrogens; and wherein R is hydrogen.

6. The pyridazine compound (I) for use to any of the claims 1-5, wherein A is selected from a group CR²=CR²-CR²=CR², thus forming either a benzopyridazine (phthalazine) wherein 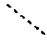 as part of the pyridazine ring denotes a double bond and wherein 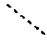 as part of ring A denotes a single bond, and wherein X denotes N, and wherein one R² is selected from the group consisting of H, D, F, Cl, CF₃, and the remaining R² are hydrogens; and wherein R is hydrogen.

7. The pyridazine compound (I) for use to any of the claims 1-6, wherein A is selected from a group CR²=CR²-CR²=CR², thus forming either a benzopyridazine (phthalazine) wherein 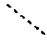 as part of the pyridazine ring denotes a double bond and wherein 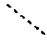 as part of ring A denotes a single bond, and wherein X denotes N, and wherein two R² are independently selected from each other from the group consisting of H, D, F, Cl, CF₃, and the remaining R² are hydrogens; and wherein R is hydrogen.

8. The pyridazine compound (I) for use according to any one of claims 1 to 7, wherein the pyridazine compound (I) is selected from the group consisting of:

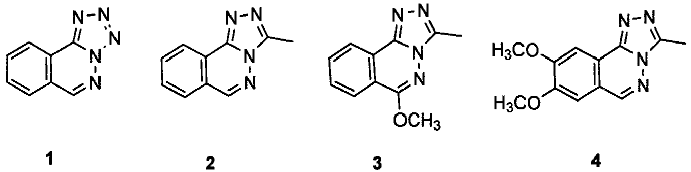

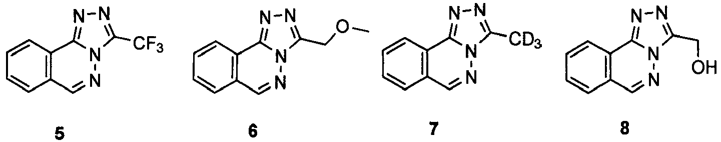

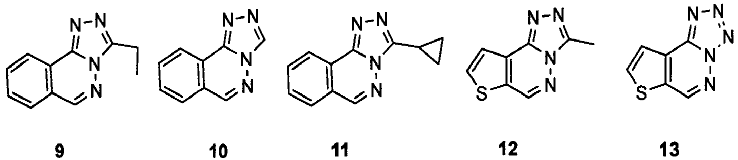

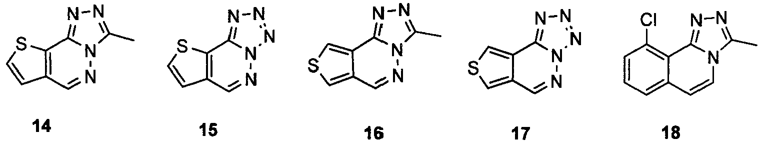

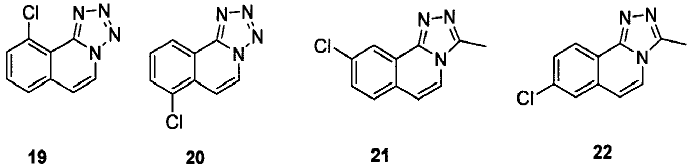

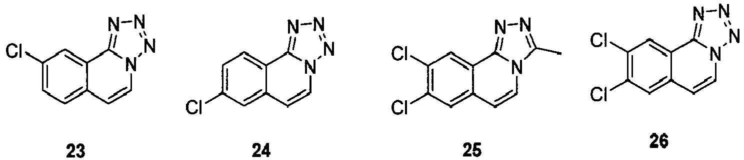

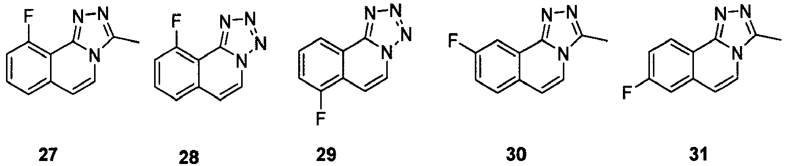

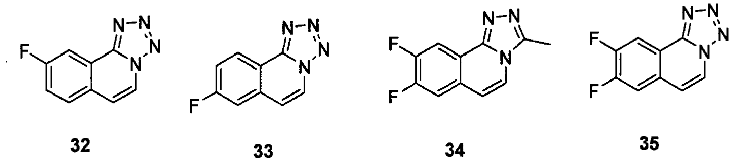

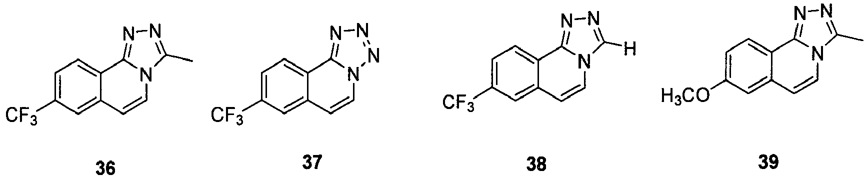

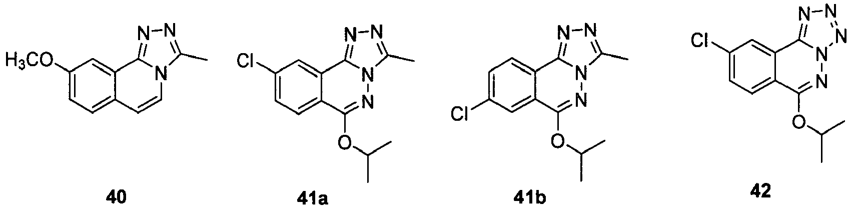

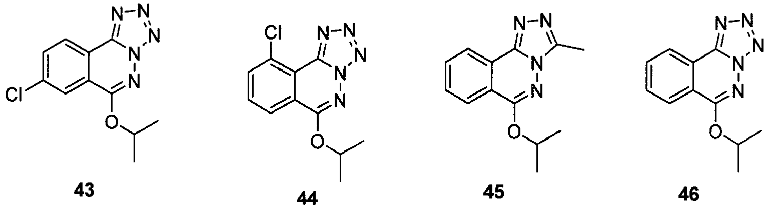

9. The pyridazine compound (I) for use to any of the claims 1-8, wherein A is selected from a group CH=CH-CH=CH, thus forming a benzopyridazine (phthalazine) wherein 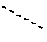 as part of the pyridazine ring denotes a double bond and wherein 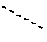 as part of ring A denotes a single bond, and wherein X denotes CCH₃ and wherein R is hydrogen.

10. The pyridazine compound (I) for use to any of the claims 1-9, wherein A is selected from a group CH=CH-CH=CH, thus forming a benzopyridazine (phthalazine) wherein 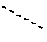 as part of the pyridazine ring denotes a double bond and wherein \ 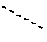 as part of ring A denotes a single bond, and wherein X denotes CH and wherein R is hydrogen.

11. The pyridazine compound (I) for use to any of the claims 1-10. That is 8-chlorotetrazolo[5,1-a]phthalazine **(24).**

12. Pharmaceutical composition comprising a pyridazine compound or a mixture of pyridazine compounds each having the structure (I) 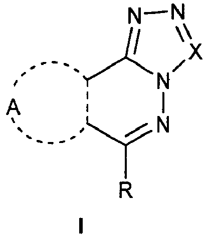 as defined in claim 1 and a pharmaceutically acceptable carrier for use in the treatment of fibrosis.

13. Pharmaceutical composition comprising a pyridazine compound or a mixture of pyridazine compounds each having the structure (I), which is used for lung fibrosis as interstitial lung diseases (ILDs) and idiopathic pulmonary fibrosis (IPF) as the most common within this group.

14. Pharmaceutical composition comprising a pyridazine compound or a mixture of pyridazine compounds each having the structure (I), which is used by inhalation for lung fibrosis.

15. Pharmaceutical composition comprising a pyridazine compound or a mixture of pyridazine compounds each having the structure (I), which in an appropriate formulation is used for the treatment of severe skin fibrosis as localized scleroderma or morphea, systemic sclerosis, PAH, hypertrophic scars, keloids, folliculitis keloidalis nuchae, cGvHD and CVI.
